# EUROPÄISCHE PATENTSCHRIFT

(11) **EP 3 299 641 B1**
(45) Veröffentlichungstag und Bekanntmachung des Hinweises auf die Patenterteilung: **22.07.2020**
(21) Anmeldenummer: 16190438.8
(22) Anmeldetag: 23.09.2016
(51) Int. Cl.: F16B 47/00

(54) **BEFESTIGUNGSVORRICHTUNG**
ATTACHMENT DEVICE
DISPOSITIF DE FIXATION

(43) Veröffentlichungstag der Anmeldung: 28.03.2018
(73) Patentinhaber: Airbus Defence and Space GmbH, 82024 Taufkirchen (DE)
(72) Erfinder: Hummel, Thomas, 88682 Salem (DE); Fetter, Viktor, 88090 Immenstaad (DE); Moitroux, Rene, 52441 Linnich (DE); Kern, Peter, 88682 Salem (DE)
(74) Vertreter: Daub, Thomas

(56) Entgegenhaltungen:
- WO-A1-96/27325
- DE-B- 1 108 820
- DE-C- 242 745
- DE-T5- 10 394 279
- FR-A1- 2 178 223
- GB-A- 191 002 995

## Beschreibung

### Stand der Technik

Die Erfindung betrifft eine Befestigungsvorrichtung.

Es ist bereits eine Befestigungsvorrichtung zu einer kraftschlüssigen Befestigung eines Objekts an einer Oberfläche, mit zumindest einem Unterdruckraum, welcher dazu vorgesehen ist, in einem Befestigungszustand an die Oberfläche anzugrenzen, mit zumindest einer den Unterdruckraum zumindest teilweise begrenzenden Dichtlippe, welche dazu vorgesehen ist, in dem Befestigungszustand an der Oberfläche anzuliegen, und mit zumindest einem Rückstellelement, welches dazu vorgesehen ist, in einem Befestigungszustand in dem Unterdruckraum einen relativen Unterdruck zu erzeugen, vorgeschlagen worden.

Ferner sind aus der DE 103 94 279 T5, der DE 242 745 C, der GB 02995 A, der WO 96/27325 A1 und der DE 11 08 820 B ebenfalls Befestigungsvorrichtungen bekannt.

Aus dem Stand der Technik sind beispielsweise bereits Befestigungen mittels Unterdruck in Form eines sogenannten "Saugnapfs" bekannt, wie beispielsweise mit einem Handtuchhaken zu einer Befestigung eines Handtuchs. Dabei handelt es sich um einen runden Napf aus einem hinreichend elastischen Material, der zum Befestigen von Objekten an glatten Oberflächen genutzt wird. Die Befestigung wird in der Regel dadurch erreicht, dass ein zentraler Unterdruckraum beim Aussetzen auf eine Oberfläche abgeschlossen und im Weiteren durch eine Kraft zusammengedrückt wird, sodass der innere Fluid teilweise oder ganz entweicht. Nach dem Entfernen der Kraft führt dies aufgrund des elastischen Saugnapfs zu einem relativen Unterdruck in dem zuvor komprimierten Unterdruckraum, was wiederum zu einem äußeren Überdruck auf den Saugnapf führt, welcher dadurch eine kraftschlüssige Verbindung mit der Oberfläche erzeugt. Der bei diesen Saugnäpfen erzeugte Unterdruck wird über eine geometrisch konvexe Fläche erzeugt.

Weiterhin sind auch Vakuumsauger bekannt, welche den Unter- bzw. den resultierenden Überdruck durch ein zusätzliches Gerät, in der Regel eine Pumpe, einmalig oder permanent erzeugen. Auch hier wird die Befestigung dadurch erreicht, dass zunächst ein zentraler Unterdruckraum beim Aussetzen auf eine Oberfläche abgeschlossen wird, allerdings im weiteren Verlauf der Unterdruckraum nicht zusammengedrückt wird, sondern zumindest teilweise ein Fluid aus dem Unterdruckraum mittels Unterdruck herausgepumpt wird.

Die Aufgabe der Erfindung besteht insbesondere darin, eine gattungsgemäße Vorrichtung mit verbesserten Eigenschaften hinsichtlich einer Funktionalität sowie hinsichtlich einer Kompaktheit bereitzustellen. Die Aufgabe wird erfindungsgemäß durch die Merkmale des Patentanspruchs 1 gelöst, während vorteilhafte Ausgestaltungen und Weiterbildungen der Erfindung den Unteransprüchen entnommen werden können.

### Vorteile der Erfindung

Die Erfindung geht aus von einer Befestigungsvorrichtung zu einer Befestigung eines Objekts an einer Oberfläche, mit zumindest einem Unterdruckraum, welcher dazu vorgesehen ist, in einem Befestigungszustand an die Oberfläche anzugrenzen, mit zumindest einer den Unterdruckraum zumindest teilweise begrenzenden Dichtlippe, welche dazu vorgesehen ist, in dem Befestigungszustand an der Oberfläche anzuliegen, und mit zumindest einem Rückstellelement, welches dazu vorgesehen ist, in einem Befestigungszustand in dem Unterdruckraum einen relativen Unterdruck zu erzeugen.

Es wird vorgeschlagen, dass die Befestigungsvorrichtung zumindest einen von dem Unterdruckraum getrennten Funktionsraum aufweist, welcher zumindest teilweise von dem Unterdruckraum umgeben ist und dazu vorgesehen ist, in einem Befestigungszustand an die Oberfläche anzugrenzen. Vorzugsweise ist die Befestigungsvorrichtung von einer Werkzeugbefestigungsvorrichtung, vorzugsweise von einer Probensammlerbefestigungsvorrichtung und besonders bevorzugt von einer Airsamplerbefestigungsvorrichtung, gebildet. Bevorzugt ist die Befestigungsvorrichtung zu einer kraftschlüssigen Befestigung eines Objekts, insbesondere eines Probensammlers, bevorzugt eines Airsampiers, an einer Oberfläche, insbesondere an einer zumindest im Wesentlichen glatten Oberfläche, vorgesehen. Vorzugsweise ist der Funktionsraum in zumindest einem Betriebszustand frei zugänglich. Bevorzugt kann das Objekt beispielsweise von einem Werkzeug gebildet sein. Unter einer "Befestigungsvorrichtung" soll in diesem Zusammenhang insbesondere eine Vorrichtung verstanden werden, über welche ein Objekt an einer Oberfläche befestigt und/oder relativ zu ihr positioniert werden kann. Vorzugsweise soll darunter insbesondere eine Vorrichtung verstanden werden, welche dazu vorgesehen ist, eine Befestigungsmöglichkeit an einer Oberfläche für ein Objekt bereitzustellen. Ferner soll in diesem Zusammenhang unter einem "Unterdruckraum" insbesondere ein Raum und/oder ein Volumen verstanden werden, welcher/welches in zumindest einem Betriebszustand, insbesondere in einem Befestigungszustand der Befestigungsvorrichtung, abgeschlossen ausgebildet ist. Vorzugsweise soll darunter insbesondere ein Raum und/oder ein Volumen verstanden werden, in welchem in zumindest einem Betriebszustand, insbesondere in einem Befestigungszustand der Befestigungsvorrichtung, ein relativer Unterdruck gegenüber einer Umgebung herrscht. Bevorzugt ist der Unterdruckraum in einem Befestigungszustand von zumindest einer Seite durch die Oberfläche begrenzt. Besonders bevorzugt ist der Unterdruckraum zumindest teilweise zu einer Erzeugung einer Haltkraft zu einer Befestigung der Befestigungsvorrichtung an der Oberfläche vorgesehen. Des Weiteren soll in diesem Zusammenhang unter einer "Dichtlippe" insbesondere ein Element verstanden werden, welches dazu vorgesehen ist, ungewollte Stoffübergänge von einem Raum, insbesondere einer Umgebung, in einen anderen Raum, insbesondere dem Unterdruckraum, zu verhindern oder zumindest zu begrenzen. Vorzugsweise soll darunter insbesondere ein Element verstanden werden, welches dazu vorgesehen ist, ein Austausch von Fluiden zwischen Räumen zu verhindern oder zumindest zu begrenzen. Bevorzugt soll darunter insbesondere ein Element verstanden werden, welches dazu vorgesehen ist, einen Übergangsbereich zwischen zwei Elementen und/oder Bauteilen, insbesondere zwischen der Befestigungsvorrichtung und der Oberfläche, insbesondere frei von einem Stoffschluss, zumindest im Wesentlichen zu verschließen. Besonders bevorzugt soll darunter insbesondere eine elastische Lippe verstanden werden, welche zumindest in einem Befestigungszustand dazu vorgesehen ist, an der Oberfläche anzuliegen und insbesondere den Unterdruckraum zumindest teilweise zu begrenzen. Unter einem "Rückstellelement" soll in diesem Zusammenhang insbesondere ein Element verstanden werden, welches dazu vorgesehen ist, zumindest in einem Befestigungszustand in dem Unterdruckraum einen relativen Unterdruck zu erzeugen. Bevorzugt soll darunter insbesondere ein mechanisches Rückstellelement verstanden werden. Vorzugsweise soll darunter insbesondere ein Element verstanden werden, welches dazu vorgesehen ist, zu einer Erzeugung des relativen Unterdrucks, ein Volumen des abgeschlossenen Unterdruckraums zu vergrößern und/oder zumindest einen Teil eines Fluids aus dem abgeschlossenen Unterdruckraum zu entfernen. Besonders bevorzugt ist das Rückstellelement dazu vorgesehen, die Befestigungsvorrichtung zumindest teilweise von der Oberfläche wegzudrücken um ein Volumen des abgeschlossenen Unterdruckraums zu vergrößern und damit einen relativen Unterdruck in dem abgeschlossenen Unterdruckraum zu erzeugen. Der relative Unterdruck in dem abgeschlossenen Unterdruckraum führt insbesondere zu einem äußeren Überdruck auf die Befestigungsvorrichtung, insbesondere auf die zumindest eine Dichtlippe, welche dadurch eine kraftschlüssige Verbindung mit der Oberfläche erzeugt. Das Rückstellelement kann dabei sowohl von einem separaten Element gebildet sein, als auch einstückig mit einem weiteren Bauteil ausgebildet sein. Dabei soll unter einer "zumindest im Wesentlichen glatten Oberfläche" insbesondere eine Oberfläche mit einer Rauheit Rz von maximal 1000 µm, vorzugsweise von maximal maximal 500 µm und besonders bevorzugt von maximal maximal 100 µm verstanden werden.

Unter einem "Funktionsraum" soll in diesem Zusammenhang insbesondere ein von einem Unterdruckraum verschiedener Raum der Befestigungsvorrichtung verstanden werden. Vorzugsweise soll darunter insbesondere ein Raum verstanden werden, welche zu einer direkten Nutzung, insbesondere durch das zu befestigende Objekt vorgesehen ist. Das zu befestigende Objekt kann dabei grundsätzlich zu einem beliebigen Zeitpunkt angeordnet oder auch gewechselt werden. Vorzugsweise soll darunter insbesondere ein Raum verstanden werden, welcher zu einer direkten Bereitstellung eines Zugangs zu der Oberfläche vorgesehen ist. Bevorzugt ist der Raum zu einer direkten Bereitstellung eines geschützten Zugangs zu der Oberfläche vorgesehen. Besonders bevorzugt ist der Funktionsraum zumindest teilweise gegenüber einer Umgebung abschirmbar. Vorzugsweise ist der Funktionsraum in einem Befestigungszustand der Befestigungsvorrichtung in zumindest eine Richtung direkt von der Oberfläche begrenzt. Ferner soll in diesem Zusammenhang unter "zumindest teilweise umgeben" insbesondere verstanden werden, dass der Funktionsraum in zumindest einer Ebene, insbesondere ausgehend von einem geometrischen Mittelpunkt, in einem Winkelbereich von zumindest 120°, vorzugsweise von zumindest 180°, bevorzugt von zumindest 270° und besonders bevorzugt von 360° von dem Unterdruckraum umschlossen wird. Vorzugsweise soll darunter insbesondere verstanden werden, dass zumindest eine gedachte Gerade existiert, welche entlang ihrer Erstreckung erst den Unterdruckraum, anschließend den Funktionsraum und zuletzt nochmals den Unterdruckraum schneidet. Unter "vorgesehen" soll insbesondere speziell ausgelegt und/oder ausgestattet verstanden werden. Darunter, dass ein Objekt zu einer bestimmten Funktion vorgesehen ist, soll insbesondere verstanden werden, dass das Objekt diese bestimmte Funktion in zumindest einem Anwendungs- und/oder Betriebszustand erfüllt und/oder ausführt.

Durch die erfindungsgemäße Ausgestaltung der Befestigungsvorrichtung kann insbesondere eine vorteilhafte Befestigung eines Objekts an einer Oberfläche bereitgestellt werden. Ferner kann dadurch vorteilhaft ein Funktionsraum bereitgestellt werden. Es kann insbesondere ein vorteilhaft geschützter Funktionsraum bereitgestellt werden. Hierdurch kann insbesondere eine vorteilhaft hohe Funktionalität sowie eine vorteilhafte Kompaktheit der Befestigungsvorrichtung bereitgestellt werden. Die Befestigungsvorrichtung kann daher insbesondere der Art gestaltet werden, dass die zur Erzeugung des Unterdrucks benötigte Fläche der Oberfläche nicht die gesamte Oberfläche, auf die die Vorrichtung angebracht wird, abdeckt. Nach Aufbringen der Vorrichtung ist somit ein Teil der Oberfläche frei erreichbar bzw. zugänglich. Diese ist insbesondere zentral angeordnet. Der Vorteil der Erfindung liegt zudem zumindest teilweise in der guten Zugänglichkeit des von der Befestigungsvorrichtung gebildeten Funktionsraums, welcher zumindest teilweise von dem Unterdruckraum umschlossen ist, und der den Funktionsraum begrenzenden Oberfläche. Ferner liegt der Vorteil gegenüber einem Vakuumsauger darin, dass kein weiterer Apparat bzw. Gerät oder eine Maschine zur Erzeugung eines Unterdrucks benötigt wird.

Ferner wird vorgeschlagen, dass der zumindest eine Unterdruckraum geometrisch unkonvex ausgebildet ist. Vorzugsweise weist der zumindest eine Unterdruckraum eine nichtkonvexe Menge auf. Unter "geometrisch unkonvex" soll in diesem Zusammenhang insbesondere verstanden werden, dass zumindest zwei Punkte innerhalb des Unterdruckraums existieren, deren direkte Verbindungsstrecke zumindest teilweise außerhalb des Unterdruckraums liegt. Der Unterdruckraum weist daher insbesondere zumindest eine Einbuchtung und/oder Aussparung auf. Vorzugsweise liegt der Funktionsraum innerhalb der Einbuchtung und/oder Aussparung. Dadurch kann insbesondere eine vorteilhafte Befestigung eines Objekts an einer Oberfläche bereitgestellt werden. Vorzugsweise kann dadurch insbesondere ein vorteilhafter Funktionsraum bereitgestellt werden. Es kann insbesondere ein vorteilhaft geschützter Funktionsraum bereitgestellt werden.

Des Weiteren wird vorgeschlagen, dass die Befestigungsvorrichtung zumindest eine zweite den Unterdruckraum zumindest teilweise begrenzende Dichtlippe aufweist, welche von der zumindest einen Dichtlippe differierend ausgebildet ist und dazu vorgesehen ist, in dem Befestigungszustand an der Oberfläche anzuliegen. Vorzugsweise ist die zumindest eine zweite Dichtlippe räumlich getrennt von der zumindest einen Dichtlippe angeordnet. Die Dichtlippen können grundsätzlich materiell, insbesondere über weitere Elemente der Befestigungsvorrichtung verbunden ausgebildet sein. Bevorzugt ist die zumindest eine zweite Dichtlippe dazu vorgesehen, den Unterdruckraum zu einer zu der zumindest einen Dichtlippe differierenden Seite hin zu begrenzen. Besonders bevorzugt sind die Dichtlippen auf gegenüberliegenden Seiten des Unterdruckraums angeordnet. Bevorzugt sind die Dichtlippen dazu vorgesehen, gemeinsam den Unterdruckraum zu begrenzen, insbesondere zu umschließen. Vorzugsweise weisen die Dichtlippen einen zumindest im Wesentlichen identischen Querschnitt auf. Dadurch kann in dem Befestigungszustand insbesondere eine zuverlässige Abdichtung des Unterdruckraums erreicht werden. Es kann insbesondere eine vorteilhafte Befestigung eines Objekts an einer Oberfläche bereitgestellt werden.

Es wird ferner vorgeschlagen, dass die zumindest eine zweite Dichtlippe in dem Befestigungszustand dazu vorgesehen ist, den zumindest einen Unterdruckraum von dem zumindest einen Funktionsraum zu trennen. Vorzugsweise ist die zumindest eine zweite Dichtlippe zumindest teilweise zwischen dem Unterdruckraum und dem Funktionsraum angeordnet. Bevorzugt ist die zumindest eine zweite Dichtlippe dazu vorgesehen, in einem Befestigungszustand einen Übergangsbereich zwischen dem Unterdruckraum und dem Funktionsraum abzudichten oder lokal zu bregrenzen. Besonders bevorzugt ist der Funktionsraum zumindest teilweise von der zumindest einen zweiten Dichtlippe umgeben. Bevorzugt ist der Funktionsraum in einem an die Oberfläche angrenzenden Bereich insbesondere vollständig von der zumindest einen zweiten Dichtlippe umgeben. Dadurch kann insbesondere eine vorteilhaft zuverlässige Trennung des Unterdruckraums von dem Funktionsraum erreicht werden. Es können zuverlässig Stoffübergänge vermieden werden. Bei einer vollständigen Umgebung des Funktionsraums durch die zumindest eine zweite Dichtlippe kann insbesondere eine zuverlässige Abdichtung des Funktionsraums gegenüber einer Umgebung erreicht werden. Wenn die Befestigungsvorrichtung zumindest im Wesentlichen gasdicht mit dem Objekt, insbesondere einem Probensammler, verbunden ist, lässt sich die Befestigungsvorrichtung dadurch insbesondere auch als Dichtung verwenden, wobei der Druck dem Umgebungsdruck entspricht. Auf diese Weise ist das Problem einer hohen Anpresskraft über einen langen Zeitraum gelöst, da die zeitlich begrenzte kraftschlüssige Verbindung das Objekt, insbesondere einen Probensammler, von sich aus auf der Stelle hält. Ein weiterer Vorteil ist, dass unmittelbar offensichtlich ist, ob die Dichtungen funktionstüchtig ist oder nicht. Es ist kein Funktionstest notwendig. Ist die Befestigungsvorrichtung nicht dicht, wird ein Druckausgleich stattfinden und am Ende der Messung lässt sich das Objekt, insbesondere einen Probensammler, ohne besonderen Kraftaufwand entfernen. Dadurch weiß der Benutzer, dass er die Anwendung wiederholen muss. Wenn dies nicht der Fall ist, und die Saugwirkung am Ende des Versuchs immer noch vorhanden ist, impliziert das zumindest im Wesentlichen die Dichtheit der Befestigungsvorrichtung und die Gültigkeit/Fehlerfreiheit der Anwendung.

Erfindungsgemäß wird vorgeschlagen, dass das zumindest eine Rückstellelement einstückig mit der zumindest einen Dichtlippe ausgebildet ist. Unter "einstückig" soll insbesondere zumindest stoffschlüssig verbunden verstanden werden, beispielsweise durch einen Schweißprozess, einen Klebeprozess, einen Anspritzprozess und/oder einen anderen, dem Fachmann als sinnvoll erscheinenden Prozess, und/oder vorteilhaft in einem Stück geformt verstanden werden, wie beispielsweise durch eine Herstellung aus einem Guss und/oder durch eine Herstellung in einem Ein- oder Mehrkomponentenspritzverfahren und vorteilhaft aus einem einzelnen Rohling.

Vorzugsweise ist die zumindest eine Dichtlippe in zumindest einem Teilbereich derart elastisch ausgebildet, dass sie als Rückstellelement fungiert. Die zumindest eine Dichtlippe bildet das Rückstellelement aus. Ausserhalb des durch die Patentansprüche bestimmten Schutzbereichs wäre jedoch auch denkbar, dass das Rückstellelement beispielsweise in die Dichtlippe eingegossen ist. Dadurch kann insbesondere eine vorteilhaft kompakte Bauweise erreicht werden. Vorzugsweise kann eine Anzahl von Bauteilen gering gehalten werden.

Ausserhalb des durch die Patentansprüche bestimmten Schutzbereichs wäre weiterhin denkbar, dass das zumindest eine Rückstellelement von einem separaten Federelement gebildet ist. Vorzugsweise bildet das Rückstellelement ein zusätzliches Rückstellelement. Unter einem "Federelement" soll insbesondere ein makroskopisches Element verstanden werden, das zumindest eine Erstreckung aufweist, die in einem normalen Betriebszustand um zumindest 10%, insbesondere um wenigstens 20%, vorzugsweise um mindestens 30% und besonders vorteilhaft um zumindest 50% elastisch veränderbar ist, und das insbesondere eine von einer Veränderung der Erstreckung abhängige und vorzugsweise durch die Veränderung bedingte Gegenkraft erzeugt, die der Veränderung entgegenwirkt. Unter einer "Erstreckung" eines Elements soll insbesondere ein maximaler Abstand zweier Punkte einer senkrechten Projektion des Elements auf eine Ebene verstanden werden. Unter einem "makroskopischen Element" soll insbesondere ein Element mit einer Erstreckung von zumindest 1 mm, insbesondere von wenigstens 5 mm und vorzugsweise von mindestens 10 mm verstanden werden. Es sind verschiedene, einem Fachmann als sinnvoll erscheinende Federelemente denkbar, wie beispielsweise axiale Einzelfedern, Spiralfedern, Tellerfederelemente, Wellrohre, Wellfedern, hydraulische Federelemente und/oder pneumatische Federelemente. Dadurch kann eine vorteilhaft hohe Rückstellkraft des Rückstellelements erreicht werden. Ferner kann dadurch ein vorteilhaft zuverlässiges Rückstellelement bereitgestellt werden.

Ferner wird vorgeschlagen, dass der zumindest eine Funktionsraum in einem Befestigungszustand gegenüber einer Umgebung abgedichtet ist. Vorzugsweise ist der zumindest eine Funktionsraum in einem Befestigungszustand zumindest teilweise mittels des Unterdruckraums gegenüber einer Umgebung abgedichtet. Unter "abdichten" soll in diesem Zusammenhang insbesondere verstanden werden, dass ein ungewollter Stoffübergang von einem Raum in einen anderen verhindert oder zumindest begrenzt wird. Vorzugsweise soll darunter insbesondere verstanden werden, dass ein Austausch von Fluiden zwischen Räumen verhindert oder zumindest begrenzt wird. Dadurch kann insbesondere eine vorteilhafte Nutzung des Funktionsraums erreicht werden. Vorzugsweise können vorteilhaft Störeinflüsse vermieden werden. Insbesondere kann dadurch eine gasdichte Trennung eines Teilbereichs der Oberfläche gegenüber einer Umgebung erreicht werden.

Des Weiteren wird vorgeschlagen, dass ein Druck in dem zumindest einen Funktionsraum eingestellt werden kann. Vorzugsweise kann aktiv ein Unter- und/oder Überdruck in dem zumindest einen Funktionsraum eingestellt werden. Hierdurch kann der zumindest eine Funktionsraum vorteilhaft an verschiedene Anforderungen und Anwendungen angepasst werden. Es kann eine vorteilhafte Nutzung des Funktionsraums erreicht werden.

Es wird ferner vorgeschlagen, dass die Befestigungsvorrichtung einen Grundkörper aufweist, an welchem die zumindest eine Dichtlippe angeordnet ist und welche zumindest ein Montageelement zu einer direkten Montage des Objekts aufweist. Vorzugsweise ist der Grundkörper zumindest teilweise ringförmig ausgebildet. Unter einem "Montageelement" soll in diesem Zusammenhang insbesondere ein Element verstanden werden, welches zumindest eine Verbindungshälfte einer insbesondere lösbaren Verbindung bereitstellt. Vorzugsweise soll darunter insbesondere ein Element verstanden werden, welches zu einer direkten Verbindung, insbesondere Kopplung, mit einem zu befestigenden Objekt vorgesehen ist. Bevorzugt erfolgt eine Verbindung mit dem zu befestigenden Objekt zumindest teilweise kraftschlüssig und/oder bevorzugt zumindest teilweise formschlüssig. Dabei soll unter "formschlüssig" insbesondere verstanden werden, dass aneinander liegende Flächen von miteinander formschlüssig verbundenen Bauteilen eine in Normalenrichtung der Flächen wirkende Haltekraft aufeinander ausüben. Insbesondere befinden sich die Bauteile in einem geometrischen Eingriff miteinander. Dadurch kann insbesondere eine vorteilhafte Montage des Objekts erreicht werden. Es kann insbesondere eine zuverlässige Verbindung zwischen dem Objekt und der Befestigungsvorrichtung bereitgestellt werden. Dadurch kann die Befestigungsvorrichtung insbesondere als kraftschlüssige Montage-und/oder Trägerplattform für verschiedene Anwendungen verwendet werden.

Es wird weiter vorgeschlagen, dass das zumindest eine Montageelement des Grundkörpers zumindest teilweise von einem Gewinde gebildet ist. Vorzugsweise ist das zumindest eine Montageelement des Grundkörpers zumindest teilweise von einem Innengewinde gebildet. Bevorzugt ist das zumindest eine Montageelement zumindest teilweise in dem Funktionsraum angeordnet. Vorzugsweise ist das zumindest eine Montageelement an einer Innenseite des Grundkörpers angeordnet. Das zumindest eine Montageelement ist insbesondere auf einer Innenseite des ringförmigen Grundkörpers angeordnet. Alternativ wäre jedoch auch denkbar, dass das zumindest eine Montageelement auf einer Außenseite des ringförmigen Grundkörpers angeordnet ist. Dadurch kann insbesondere ein vorteilhaft zuverlässiges Montageelement bereitgestellt werden. Es kann insbesondere ein Montageelement bereitgestellt werden, an welchem eine schnelle und einfache Montage des Objekts möglich ist.

Ferner geht die Erfindung aus von einem Verfahren zu einer Montage und/oder einem Betrieb der Befestigungsvorrichtung. Es wird vorgeschlagen, dass die Befestigungsvorrichtung in einem ersten Schritt mittels einer Kraft, insbesondere einer externen Kraft, gegen die Oberfläche gedrückt wird, sodass sich ein Volumen des zumindest einen Unterdruckraums verringert und das zumindest eine Rückstellelement ausgelenkt wird. Vorzugsweise wird die externe Kraft von einem Benutzer aufgebracht, welcher die Befestigungsvorrichtung montiert. Vorzugsweise wird dabei das zumindest eine Rückstellelement elastisch, insbesondere federelastisch, ausgelenkt. Dadurch kann eine vorteilhaft einfache und schnelle Montage der Befestigungsvorrichtung erreicht werden. Es kann insbesondere eine Montage der Befestigungsvorrichtung frei von Hilfsmitteln erreicht werden.

Des Weiteren wird vorgeschlagen, dass das zumindest eine Rückstellelement in einem weiteren Schritt eine entgegen der Kraft gerichtete Rückstellkraft erzeugt, welche einen relativen Unterdruck in dem zumindest einen Unterdruckraum erzeugt. Vorzugsweise erzeugt das zumindest eine Rückstellelement in einem weiteren Schritt, nachdem die Kraft entfernt wurde, eine entgegen der Kraft gerichtete Rückstellkraft, welche einen relativen Unterdruck in dem zumindest einen Unterdruckraum erzeugt. Bevorzugt ist das zumindest eine Rückstellelement von einem elastischen Element gebildet. Unter einem "elastischen Element" soll insbesondere ein Element verstanden werden, das wiederholt verformbar ist, ohne dass dadurch das Element mechanisch beschädigt oder zerstört wird, und das insbesondere nach einer Verformung selbstständig wieder einer Grundform zustrebt. Vorzugsweise soll darunter insbesondere ein Element verstanden werden, welches bei einer Verformung insbesondere eine von der Verformung abhängige und vorzugsweise zu der Verformung proportionale Gegenkraft erzeugt, die der Verformung entgegen wirkt. Dadurch kann eine vorteilhaft einfache und schnelle kraftschlüssige Befestigung der Befestigungsvorrichtung auf der Oberfläche erreicht werden. Es kann insbesondere eine Montage der Befestigungsvorrichtung frei von Hilfsmitteln erreicht werden.

Es wird ferner vorgeschlagen, dass die Durchführung unter Bedingungen reduzierter Schwerkraft erfolgt. Vorzugsweise soll dieses Verfahren im Weltraum angewandt werden, wie beispielsweise bei µg in einem Raumschiff, einem Prozess in einem Raumschiff bei Beschleunigungen von 10⁻⁶ xg bis 10 xg, auf einem Planeten, wie dem Mars, und/oder auf einem Trabanten, wie dem Mond. Die g-Werte sind dabei insbesondere auf einem Planeten und/oder einem Asteroiden oder in einem fliegenden Raumschiff zu verstehen. Unter "Bedingungen reduzierter Schwerkraft" sollen insbesondere Bedingungen verstanden werden, bei denen eine Schwerewirkung von maximal 0,9 xg, vorteilhaft von minimal bis 1*10⁻³ xg, vorzugsweise von minimal bis 1*10⁻⁶ xg und besonders bevorzugt von minimal bis 1*10⁻⁸ xg, wirksam ist. Dadurch kann eine vorteilhafte Anwendung des Verfahrens erreicht werden. Es kann insbesondere eine sinnvolle und zuverlässige Befestigungsvorrichtung für die Verwendung unter Bedingungen reduzierter Schwerkraft bereitgestellt werden.

Es wird weiter vorgeschlagen, dass die Oberfläche von einer Hautfläche gebildet ist. Unter einer "Hautfläche" soll in diesem Zusammenhang insbesondere die Oberfläche der Haut eines Lebewesens, insbesondere eines Menschen, verstanden werden. Dadurch kann insbesondere die temporäre Befestigung von Objekten an einem Körper ermöglicht werden. Es kann insbesondere eine kraftschlüssige Befestigung an einem Körper ermöglicht werden.

Ferner wird vorgeschlagen, dass mittels der Befestigungsvorrichtung ein Muster auf der Oberfläche erzeugt wird. Vorzugsweise wird mittels der Befestigungsvorrichtung ein Muster auf der Hautfläche erzeugt. Das Muster kann dabei sowohl von einem Sugillationsmuster gebildet sein, welches in dem Unterdruckraum entsteht, als auch von einem Aufdruck mittels Farbe, welcher in dem Funktionsraum aufgebracht wird. Dadurch kann insbesondere eine frei wählbare Lifestyle-Zeichnung auf der Haut und/oder einer anderen Oberfläche erzeugt werden. Insbesondere kann durch die Befestigungsvorrichtung eine Positionsgenauigkeit des Musters gewährleistet werden.

Des Weiteren wird vorgeschlagen, dass in einem Verfahrensschritt auf einen den Funktionsraum begrenzenden Bereich der Oberfläche eine Substanz auf die Oberfläche aufgebracht wird. Vorzugsweise wird die Substanz auf eine von einer Hautfläche gebildete Oberfläche aufgebracht. Es sind verschiedene, einem Fachmann als sinnvoll erscheinende Substanzen denkbar, wie beispielsweise Salben, Medikamente, Pflegemittel, lokale oder biochemisch funktionale Indikatoren und/oder funktionale Marker. Dadurch kann insbesondere ein geschützter Bereich zu einem Aufbringen von Substanzen bereitgestellt werden. Hierdurch kann insbesondere eine vorteilhafte medizinische Anwendung der Befestigungsvorrichtung bereitgestellt werden.

Es wird ferner vorgeschlagen, dass in einem Verfahrensschritt die auf den, den Funktionsraum begrenzenden Bereich der Oberfläche aufgebrachte Substanz mittels des Objekts gemessen wird. Vorzugsweise wird die auf den, den Funktionsraum begrenzenden Bereich der Oberfläche aufgebrachte Substanz mittels des Objekts optisch, elektrisch und/oder auf eine andere, einem Fachmann als sinnvoll erscheinende Art gemessen. Grundsätzlich wäre jedoch auch eine andere, einem Fachmann als sinnvoll erscheinende Messmethode denkbar. Ferner wäre zudem denkbar, dass über das Objekt eine Veränderung des den Funktionsraum begrenzenden Bereichs der Oberfläche gemessen, insbesondere optisch gemessen, wird. Hierdurch könnten beispielsweise auf einer Hautfläche Hautreaktionen auf Substanzen, insbesondere ohne äußere Störfaktoren gemessen werden. Ferner können dadurch vorteilhaft geschützte Messungen durchgeführt werden.

Die erfindungsgemäße Befestigungsvorrichtung sowie das Verfahren sollen hierbei nicht auf die oben beschriebene Anwendung und Ausführungsform beschränkt sein. Insbesondere können die erfindungsgemäße Befestigungsvorrichtung sowie das Verfahren zu einer Erfüllung einer hierin beschriebenen Funktionsweise eine von einer hierin genannten Anzahl von einzelnen Elementen, Bauteilen und Einheiten abweichende Anzahl aufweisen.

### Zeichnungen

Weitere Vorteile ergeben sich aus der folgenden Zeichnungsbeschreibung. In den Zeichnungen sind sechs Ausführungsbeispiele der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: eine an einer Oberfläche befestigte erfindungsgemäße Befestigungsvorrichtung und ein an der Befestigungsvorrichtung montiertes Objekt in einer schematischen Darstellung,
- Fig. 2: die erfindungsgemäße Befestigungsvorrichtung und das an der Befestigungsvorrichtung montierte Objekt in einer schematischen Draufsicht,
- Fig. 3: die erfindungsgemäße Befestigungsvorrichtung und das an der Befestigungsvorrichtung montierte Objekt in einem Montagezustand in einer schematischen Schnittdarstellung entlang der Schnittlinie III-III,
- Fig. 4: die erfindungsgemäße Befestigungsvorrichtung und das an der Befestigungsvorrichtung montierte Objekt in einem Befestigungszustand in einer schematischen Schnittdarstellung entlang der Schnittlinie III-III,
- Fig. 5: ein schematisches Ablaufdiagramm eines Verfahrens zu einer Montage der Befestigungsvorrichtung,
- Fig. 6: eine alternative erfindungsgemäße Befestigungsvorrichtung und ein alternatives an der Befestigungsvorrichtung montiertes Objekt in einem Befestigungszustand in einer schematischen Schnittdarstellung,
- Fig. 7: eine weitere alternative erfindungsgemäße Befestigungsvorrichtung und ein weiteres alternatives an der Befestigungsvorrichtung montiertes Objekt in einem Befestigungszustand in einer schematischen Schnittdarstellung,
- Fig. 8: eine weitere alternative erfindungsgemäße Befestigungsvorrichtung und ein weiteres alternatives an der Befestigungsvorrichtung montiertes Objekt in einem Befestigungszustand in einer schematischen Draufsicht,
- Fig. 9: die weitere alternative erfindungsgemäße Befestigungsvorrichtung und das an der Befestigungsvorrichtung montierte Objekt in einem Befestigungszustand in einer schematischen Schnittdarstellung entlang der Schnittlinie IX-IX,
- Fig. 10: eine weitere alternative erfindungsgemäße Befestigungsvorrichtung und ein an der Befestigungsvorrichtung montierte Objekt in einem Montagezustand in einer schematischen Schnittdarstellung,
- Fig. 11: die weitere alternative erfindungsgemäße Befestigungsvorrichtung und das an der Befestigungsvorrichtung montierte Objekt in einem Befestigungszustand in einer schematischen Schnittdarstellung,
- Fig. 12: eine weitere alternative erfindungsgemäße Befestigungsvorrichtung und ein an der Befestigungsvorrichtung montierte Objekt in einem Montagezustand in einer schematischen Schnittdarstellung und
- Fig. 13: die weitere alternative erfindungsgemäße Befestigungsvorrichtung und das an der Befestigungsvorrichtung montierte Objekt in einem Befestigungszustand in einer schematischen Schnittdarstellung.

### Beschreibung der Ausführungsbeispiele

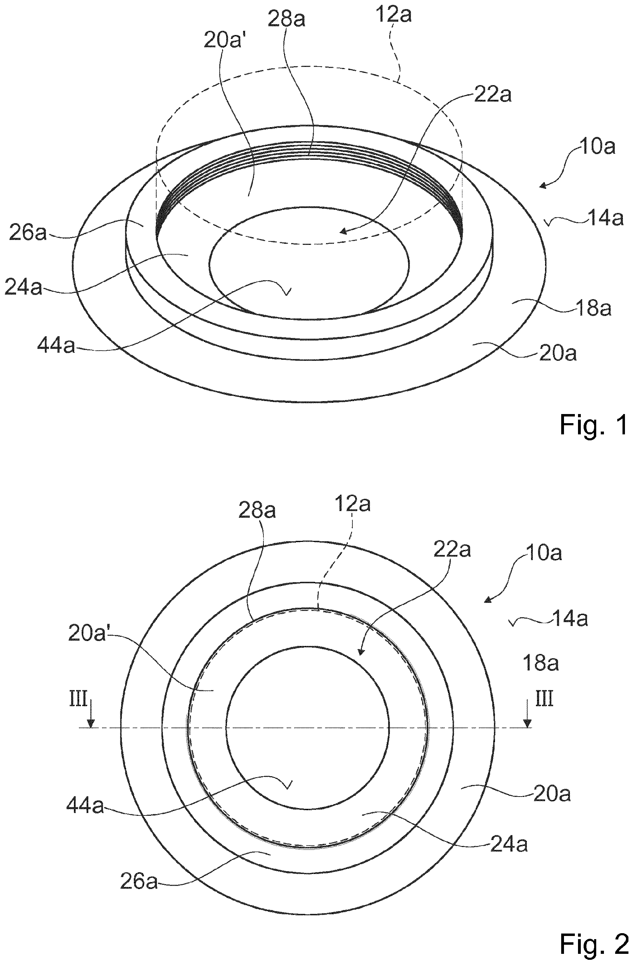

Figur 1 zeigt eine Befestigungsvorrichtung 10a. Die Befestigungsvorrichtung 10a ist zu einer Befestigung eines Objekts 12a an einer Oberfläche 14a vorgesehen. Die Befestigungsvorrichtung 10a ist zu einer kraftschlüssigen Befestigung eines Objekts 12a an einer Oberfläche 14a vorgesehen. Die Befestigungsvorrichtung 10a ist zu einer kraftschlüssigen Befestigung eines Objekts 12a an einer glatten Oberfläche 14a vorgesehen. Eine Befestigung kann dabei unabhängig von einer Ausrichtung der Oberfläche 14a erfolgen. Die Oberfläche 14a ist von einer glatten formfesten Oberfläche 14a gebildet.

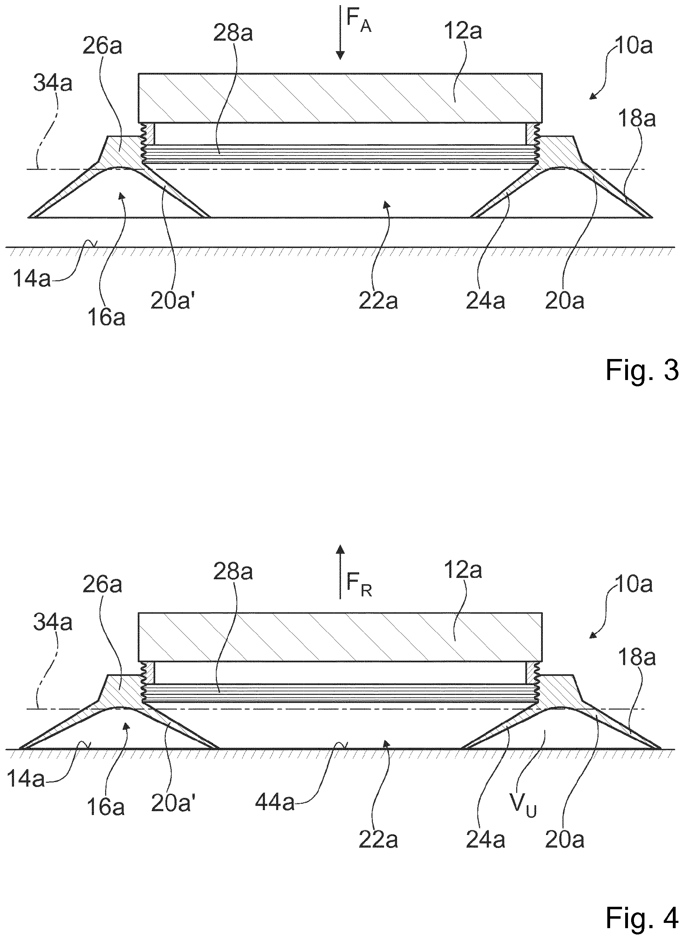

Die Befestigungsvorrichtung 10a weist einen Unterdruckraum 16a auf. Der Unterdruckraum 16a ist dazu vorgesehen, in einem Befestigungszustand an die Oberfläche 14a anzugrenzen. Der Unterdruckraum 16a grenzt in einem Befestigungszustand mit einer Seite an die Oberfläche 14a an (Figur 4). In einem nicht befestigten Zustand ist diese Seite des Unterdruckraums 16a geöffnet (Figur 3). Der Unterdruckraum 16a ist geometrisch unkonvex ausgebildet. Der Unterdruckraum 16a weist eine nichtkonvexe Menge auf. Der Unterdruckraum 16a ist im Wesentlichen ringförmig ausgebildet (Figur 2). Grundsätzlich ist jedoch auch eine andere, einem Fachmann als sinnvoll erscheinende Formgebung des Unterdruckraums 16a denkbar.

Ferner weist die Befestigungsvorrichtung 10a eine den Unterdruckraum 16a teilweise begrenzende Dichtlippe 18a auf. Die Dichtlippe 18a bildet eine erste Dichtlippe 18a. Die Dichtlippe 18a begrenzt den Unterdruckraum 16a zu einer Außenseite hin. Die Dichtlippe 18a ist dazu vorgesehen, in dem Befestigungszustand an der Oberfläche 14a anzuliegen.

Des Weiteren weist die Befestigungsvorrichtung 10a eine zweite den Unterdruckraum 16a teilweise begrenzende Dichtlippe 24a auf. Die zweite Dichtlippe 24a ist von der ersten Dichtlippe 18a differierend ausgebildet. Die zweite Dichtlippe 24a begrenzt den Unterdruckraum 16a zu einer Innenseite hin. Die zweite Dichtlippe 24a ist ebenfalls dazu vorgesehen, in dem Befestigungszustand an der Oberfläche 14a anzuliegen. Die Dichtlippen 18a, 24a sind jeweils dazu vorgesehen, den Unterdruckraum 16a an der Oberfläche 14a gegenüber einer Umgebung abzudichten. Die Befestigungsvorrichtung 10a weist ferner einen Grundkörper 26a auf. Der Grundkörper 26a ist ringförmig ausgebildet. Der Grundkörper 26a weist eine kreisförmige Außenkontur auf. Die Dichtlippen 18a, 24a sind an dem Grundkörper 26a angeordnet. Die Dichtlippen 18a, 24a sind über den Grundkörper 26a miteinander verbunden. Die Dichtlippen 18a, 24a sind räumlich getrennt voneinander angeordnet. Die Dichtlippen 18a, 24a sind dazu vorgesehen, den Unterdruckraum 16a zu gegenüberliegenden Seiten hin zu begrenzen. Die Dichtlippen 18a, 24a sind auf gegenüberliegenden Seiten des Unterdruckraums 16a angeordnet. Ferner weisen die Dichtlippen 18a, 24a einen im Wesentlichen identischen Querschnitt auf. In einer Schnittebene senkrecht zu einer Tangente des Grundkörpers 26a weisen die Dichtlippen 18a, 24a jeweils eine konische, spitz zulaufende Form auf. Die Dichtlippen 18a, 24a bilden jeweils an einem freien, dem Grundkörper 26a abgewandten Ende eine Dichtkante. Die Dichtlippen 18a, 24a sind jeweils in einer unbelasteten Stellung, wie in Figur 3 dargestellt, V-förmig zueinander angeordnet. Die Dichtlippen 18a, 24a sind dazu vorgesehen, durch die Oberfläche 14a in entgegengesetzte Richtungen elastisch verformt zu werden. Die Dichtlippen 18a, 24a sind jeweils bei einer Montage dazu vorgesehen, durch die Oberfläche 14a gegeneinander verspreizt zu werden. Ein Öffnungswinkel zwischen den beiden Dichtlippen 18a, 24a wird daher bei einer Montage vergrößert. Dabei werden die Dichtkanten der Dichtlippen 18a, 24a voneinander wegbewegt und die Dichtlippen 18a, 24a werden voneinander weggekippt. Die Dichtkanten der Dichtlippen 18a, 24a liegen dabei ständig an der Oberfläche 14a an. Die Dichtlippen 18a, 24a sind in einem Befestigungszustand dazu vorgesehen, bündig an der Oberfläche 14a anzuliegen (Figur 4).

Die Befestigungsvorrichtung 10a hat daher die Eigenschaft, dass zur Erzeugung des Unterdrucks kein zusätzlicher Apparat, kein zusätzliches Gerät oder keine zusätzliche Maschine benötigt wird. Das Abdichten erfolgt durch die zwei Dichtlippen 18a, 24a. Der Zweck der Befestigungsvorrichtung 10a ist insbesondere die Schaffung einer fixierten Basis mittels einer kraftschlüssigen Verbindung des Objektes 12a mit der Oberfläche 14a.

Ferner weist die Befestigungsvorrichtung 10a zwei Rückstellelemente 20a, 20a' auf. Die Rückstellelemente 20a, 20a' sind dazu vorgesehen, in einem Befestigungszustand in dem Unterdruckraum 16a einen relativen Unterdruck zu erzeugen. Die Rückstellelemente 20a, 20a' sind dazu vorgesehen, in einem Befestigungszustand, zu einer Erzeugung des relativen Unterdrucks, ein Volumen V_{U} des abgeschlossenen Unterdruckraum 16a zu vergrößern. Die Rückstellelemente 20a, 20a' sind hierzu in einem Befestigungszustand dazu vorgesehen, eine von der Oberfläche 14a wegweisende Kraft auf die Befestigungsvorrichtung 10a zu wirken. Der relative Unterdruck in dem abgeschlossenen Unterdruckraum 16a führt zu einem äußeren Überdruck auf die Befestigungsvorrichtung 10a, insbesondere auf die Dichtlippen 18a, 24a, welche dadurch eine kraftschlüssige Verbindung mit der Oberfläche 14a erzeugen. Die Rückstellelemente 20a, 20a' sind einstückig mit den Dichtlippen 18a, 24a ausgebildet. Die Rückstellelemente 20a, 20a' sind mit jeweils einer der Dichtlippen 18a, 24a einstückig ausgebildet. Die Dichtlippen 18a, 24a sind derart elastisch ausgebildet, dass sie als Rückstellelemente 20a, 20a' fungiert. Die Dichtlippen 18a, 24a bilden jeweils die Rückstellelemente 20a, 20a'. Grundsätzlich wäre jedoch auch denkbar, dass die Rückstellelemente 20a, 20a' beispielsweise in die Dichtlippen 18a, 24a eingegossen sind um eine vorteilhaft hohe Rückstellkraft F_{R} zu erreichen (Figur 3, 4).

Des Weiteren weist die Befestigungsvorrichtung 10a einen von dem Unterdruckraum 16a getrennten Funktionsraum 22a auf. Der Funktionsraum 22a ist zumindest in einem Befestigungszustand von dem Unterdruckraum 16a getrennt. Der Funktionsraum 22a ist zu einer direkten Nutzung durch das zu befestigende Objekt 12a vorgesehen. Der Funktionsraum 22a ist zu einer direkten Bereitstellung eines Zugangs zu der Oberfläche 14a vorgesehen. Der Funktionsraum 22a ist zumindest teilweise von dem Unterdruckraum 16a umgeben. Der Funktionsraum 22a ist in einer Ebene parallel zu der Oberfläche 14a vollständig von dem Unterdruckraum 16a umgeben. Der Funktionsraum 22a ist in einer Ebene parallel zu einer Haupterstreckungsebene 34a der Befestigungsvorrichtung 10a vollständig von dem Unterdruckraum 16a umgeben. Ferner ist der Funktionsraum 22a dazu vorgesehen, in einem Befestigungszustand an die Oberfläche 14a anzugrenzen. Der Funktionsraum 22a ist in einem Befestigungszustand gegenüber einer Umgebung abgedichtet. Der Funktionsraum 22a ist in einem Befestigungszustand bei einem montierten Objekt 12a gegenüber einer Umgebung abgedichtet. Ferner ist der Funktionsraum 22a in einem Befestigungszustand gegenüber dem Unterdruckraum 16a abgedichtet. Die zweite Dichtlippe 24a ist in dem Befestigungszustand dazu vorgesehen, den Unterdruckraum 16a von dem Funktionsraum 22a zu trennen. Die zweite Dichtlippe 24a ist zwischen dem Funktionsraum 22a und dem Unterdruckraum 16a angeordnet. Die zweite Dichtlippe 24a trennt in einem Befestigungszustand den Funktionsraum 22a von dem Unterdruckraum 16a (Figur 1, 4).

Der Grundkörper 26a der Befestigungsvorrichtung 10a weist ein Montageelement 28a auf. Das Montageelement 28a ist zu einer direkten Montage des Objekts 12a vorgesehen. Das Montageelement 28a des Grundkörpers 26a ist von einem Gewinde gebildet. Das Montageelement 28a ist von einem Innengewinde gebildet. Grundsätzlich wäre jedoch auch eine andere, einem Fachmann als sinnvoll erscheinende Ausbildung des Montageelements 28c denkbar, wie beispielsweise als Außengewinde, als Bajonettverschluss und/oder als Unterdruckhalterung. Das Montageelement 28a ist teilweise in dem Funktionsraum 22a angeordnet. Ferner ist das Montageelement 28a an einer Innenseite des Grundkörpers 26a angeordnet. Das Montageelement 28a bildet an einer Innenseite des Grundkörpers 26a das Gewinde aus. Das Gewinde des Montageelements 28a weist einen Durchmesser auf, der annähernd einem Durchmesser des Funktionsraums 22a angeordnet ist. Dadurch kann der Funktionsraum 22a vorteilhaft durch das Objekt 12a genutzt werden (Figur 1, 3,4).

Es sind verschiedene, einem Fachmann als sinnvoll erscheinende Ausbildungen des Objekts 12a denkbar. Das Objekt 12a ist von einem Werkzeug gebildet. Das Objekt 12a ist hier beispielhaft von einem Airsampler gebildet. Das Objekt 12a grenzt direkt an den Funktionsraum 22a an. Das Objekt 12a begrenzt den Funktionsraum 22a in eine von der Oberfläche 14a abgewandten Richtung. Diese Befestigungsvorrichtung 10a dient daher insbesondere als Träger für fixe oder austauschbare "Werkzeuge", z.B. einem Airsampier, einem Probenkopf eines E-Nose Systems, einer Kamera oder dergleichen, oder zum Abschluss des Innenvolumens für weitere Anwendungen.

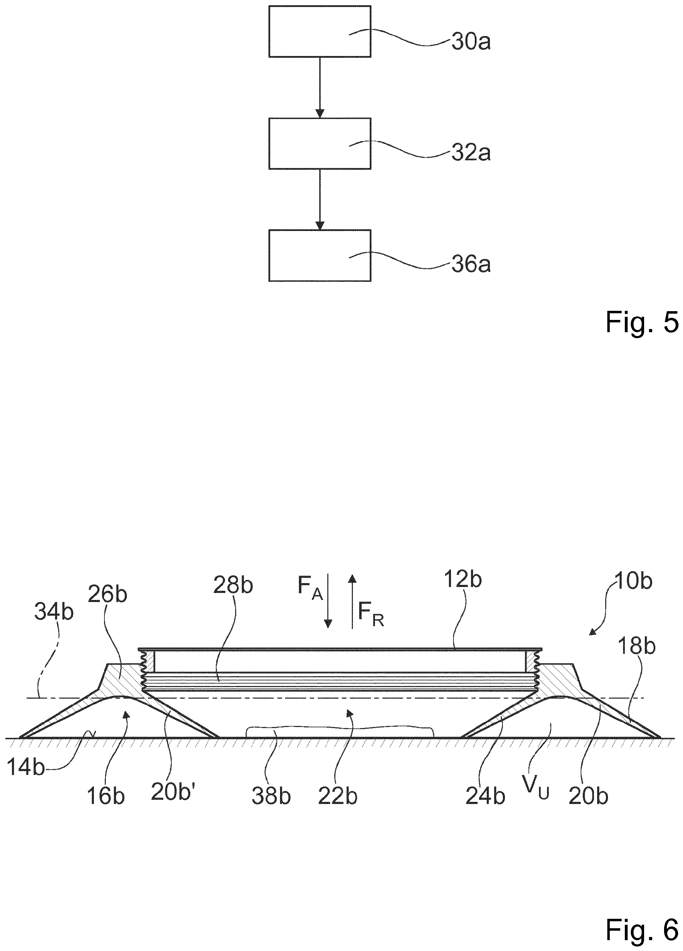

Figur 5 zeigt ein Ablaufdiagramm eines Verfahrens zu einer Montage der Befestigungsvorrichtung 10a. Bei dem Verfahren wird die Befestigungsvorrichtung 10a in einem ersten Schritt 30a mittels einer externen Kraft F_{A} gegen die Oberfläche 14a gedrückt, sodass sich ein Volumen V_{U} des Unterdruckraums 16a verringert und die Rückstellelemente 20a, 20a' ausgelenkt werden. Die externe Kraft F_{A} wird von einem Benutzer aufgebracht. Grundsätzlich wäre jedoch auch denkbar, dass die externe Kraft F_{A} von einer beliebigen Einheit, insbesondere einer externen Einheit, aufgebracht wird. Hierdurch könnte insbesondere gewährleistet werden, dass die Befestigungsvorrichtung 10a mit einer definierten Kraft F_{A} angedrückt wird. Durch das Anpressen der Befestigungsvorrichtung 10a gegen die Oberfläche 14a mit der externen Kraft F_{A} entweicht insbesondere ein Teil des Fluids aus dem Unterdruckraum 16a. Der erste Schritt 30a ist beispielhaft in der Figur 3 dargestellt. Anschließend wird die externe Kraft F_{A} entfernt. Die Rückstellelemente 20a, 20a' erzeugen nun in einem weiteren Schritt 32a eine entgegen der externen Kraft F_{A} gerichtete Rückstellkraft F_{R}, welche einen relativen Unterdruck in dem Unterdruckraum 16a erzeugt. Nach dem Entfernen der Kraft F_{A} führt dies infolge des elastischen Rückfederns der Dichtlippen 18a, 24a durch die Wirkung der Rückstellelemente 20a, 20a' zu einem relativen Unterdruck in dem Unterdruckraum 16a, was wiederum zu einem äußeren Überdruck auf die Befestigungsvorrichtung 10a führt, welche dadurch eine zumindest im wesentlichen kraftschlüssige Verbindung mit der Oberfläche 14a aufweist. Der weitere Schritt 32a ist beispielhaft in der Figur 4 dargestellt. Anschließend kann das Objekt 12a in einem weiteren Schritt 36a an dem Montageelement 28a montiert werden. Grundsätzlich wäre jedoch auch denkbar, dass das Objekt 12a bereits vor dem ersten Schritt 30a an dem Befestigungsvorrichtung 10a befestigt wird.

Eine Durchführung des Verfahrens erfolgt unter Bedingungen reduzierter Schwerkraft. Das Verfahren wird im Weltraum bei lokalen Schwerkraftswerten von 10⁻⁶ xg bis 10 xg durchgeführt. Das Verfahren wird insbesondere im Weltraum angewandt, wie beispielsweise bei µg in einem Raumschiff, einem Prozess in einem Raumschiff bei Beschleunigungen von 10⁻⁶ xg bis 10 xg, auf einem Planeten, wie dem Mars, und/oder auf einem Trabanten, wie dem Mond. Die Befestigungsvorrichtung 10a kann jedoch auch einer künstlichen Prozessbeschleunigung ausgesetzt sein, welche von den angegebenen Schwerkraftswerten abweicht.

Unter Bedingungen reduzierter Schwerkraft kann die Befestigungsvorrichtung 10a dabei insbesondere als gasdichte Trennung zwischen der Umgebung und dem den Funktionsraum 22a begrenzenden Bereich 44a der Oberfläche 14a verwendet werden.

Insbesondere unter Bedingungen reduzierter Schwerkraft kann die Befestigungsvorrichtung 10a insbesondere als zeitlich begrenzte Befestigung von zumindest einem Objekt 12a, Gerät, Apparat und/oder zumindest einer Maschine auf einer Oberfläche 14a verwendet werden.

Grundsätzlich kann das Verfahren oder ein Verfahren zu einem Betrieb der Befestigungsvorrichtung 10a auch in alternativen Bereichen angewendet werden.

In den Figuren 6 bis 13 sind fünf weitere Ausführungsbeispiele der Erfindung gezeigt. Die nachfolgenden Beschreibungen beschränken sich im Wesentlichen auf die Unterschiede zwischen den Ausführungsbeispielen, wobei bezüglich gleichbleibender Bauteile, Merkmale und Funktionen auf die Beschreibung der anderen Ausführungsbeispiele, insbesondere der Figuren 1 bis 5, verwiesen werden kann. Zur Unterscheidung der Ausführungsbeispiele ist der Buchstabe a in den Bezugszeichen des Ausführungsbeispiels der Figuren 1 bis 5 durch die Buchstaben b bis f in den Bezugszeichen der Ausführungsbeispiele der Figuren 6 bis 13 ersetzt. Bezüglich gleich bezeichneter Bauteile, insbesondere in Bezug auf Bauteile mit gleichen Bezugszeichen, kann grundsätzlich auch auf die Zeichnungen und/oder die Beschreibung der anderen Ausführungsbeispiele, insbesondere der Figuren 1 bis 5, verwiesen werden.

Figur 6 zeigt eine Befestigungsvorrichtung 10b. Die Befestigungsvorrichtung 10b ist zu einer Befestigung eines Objekts 12b an einer Oberfläche 14b vorgesehen. Die Befestigungsvorrichtung 10b ist zu einer kraftschlüssigen Befestigung eines Objekts 12b an einer Oberfläche 14b vorgesehen. Die Oberfläche 14b ist hier beispielhaft von einer Hautfläche gebildet.

Die Befestigungsvorrichtung 10b weist einen Unterdruckraum 16b auf. Ferner weist die Befestigungsvorrichtung 10b eine den Unterdruckraum 16b teilweise begrenzende Dichtlippe 18b auf. Die Dichtlippe 18b bildet eine erste Dichtlippe 18b. Des Weiteren weist die Befestigungsvorrichtung 10b eine zweite den Unterdruckraum 16b teilweise begrenzende Dichtlippe 24b auf. Die Befestigungsvorrichtung 10b weist ferner zwei Rückstellelemente 20b, 20b' auf. Die Rückstellelemente 20b, 20b' sind mit jeweils einer der Dichtlippen 18b, 24b einstückig ausgebildet. Des Weiteren weist die Befestigungsvorrichtung 10b einen von dem Unterdruckraum 16b getrennten Funktionsraum 22b auf.

Die Befestigungsvorrichtung 10b weist ferner einen Grundkörper 26b auf. Der Grundkörper 26b der Befestigungsvorrichtung 10b weist ein Montageelement 28b auf. Das Montageelement 28b ist zu einer direkten Montage des Objekts 12b vorgesehen. Das Montageelement 28b des Grundkörpers 26b ist von einem Gewinde gebildet. Das Montageelement 28b ist von einem Innengewinde gebildet. Es sind verschiedene, einem Fachmann als sinnvoll erscheinende Ausbildungen des Objekts 12b denkbar. Das Objekt 12b ist hier beispielhaft von einer Abdeckung gebildet. Das Objekt 12b ist von einem Deckel gebildet, welcher den Funktionsraum 22b temporär von einer Umgebung trennt.

Bei einem Verfahren zu einem Betrieb der Befestigungsvorrichtung 10b wird die Befestigungsvorrichtung 10b auf einer von einer Hautfläche gebildeten Oberfläche 14b befestigt. Anschließend kann in einem Verfahrensschritt auf einem den Funktionsraum 22b begrenzenden Bereich 44b der Oberfläche 14b eine Substanz 38b, wie beispielsweise eine Salbe, auf die Oberfläche 14b aufgebracht werden. Vorteilhaft kann in einem darauffolgenden Verfahrensschritt die auf den, den Funktionsraum 22b begrenzenden Bereich 44b der Oberfläche 14b aufgebrachte Substanz 38b mittels des Objekts 12b, beispielsweise optisch, gemessen werden. Anschließend kann der Funktionsraum 22b mittels des Objekts 12b von einer Umgebung getrennt werden. Dadurch kann eine Verunreinigung vermieden werden. Ferner kann erreicht werden, dass der den Funktionsraum 22b begrenzenden Bereich 44b der Oberfläche 14b temporär geschützt ist. Es kann ein unbeabsichtigtes Entfernen der Substanz 38b vermieden werden.

Eine weitere mögliche Anwendung der Befestigungsvorrichtung liegt daher in der Einbringung von Stoffen auf dem den Funktionsraum 22b begrenzenden Bereich 44b der Oberfläche 14b. Diese Stoffe können dabei beispielsweise durch das Objekt 12b auf dem Bereich 44b der Oberfläche 14b fixiert und/oder überwacht werden.

Das Objekt 12b ist von einer Messeinrichtung gebildet. Das Objekt 12b ist beispielhaft von einer optischen Messeinrichtung gebildet.

Grundsätzlich kann das Verfahren auch in alternativen Bereichen angewendet werden.

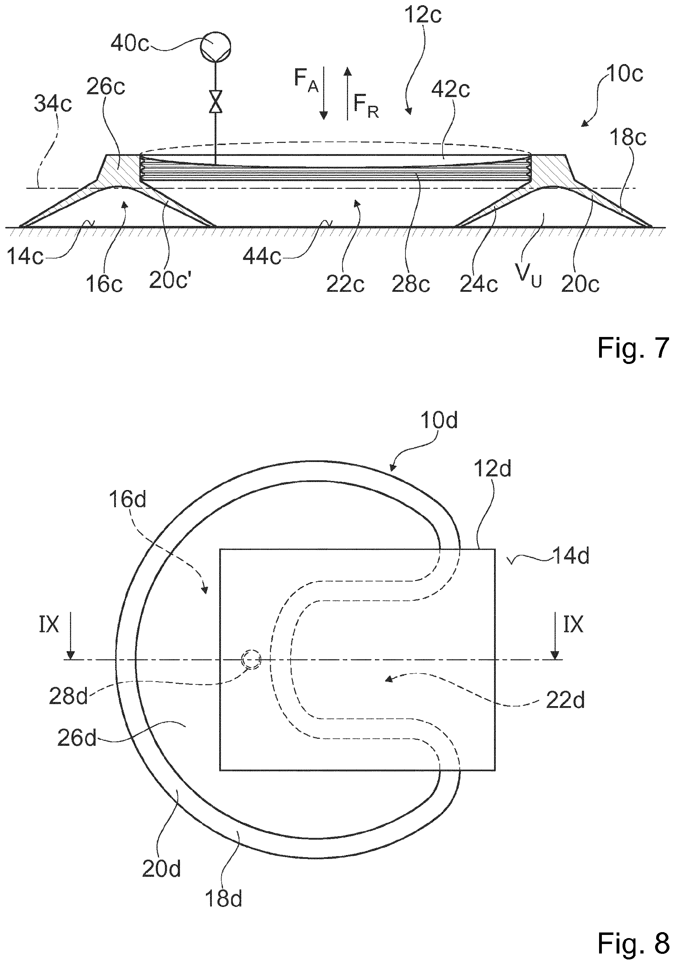

Figur 7 zeigt eine Befestigungsvorrichtung 10c. Die Befestigungsvorrichtung 10c ist zu einer Befestigung eines Objekts 12c an einer Oberfläche 14c vorgesehen. Die Befestigungsvorrichtung 10c ist zu einer kraftschlüssigen Befestigung eines Objekts 12c an einer Oberfläche 14c vorgesehen. Die Oberfläche 14c ist hier beispielhaft von einer Hautfläche gebildet.

Die Befestigungsvorrichtung 10c weist einen Unterdruckraum 16c auf. Ferner weist die Befestigungsvorrichtung 10c eine den Unterdruckraum 16c teilweise begrenzende Dichtlippe 18c auf. Die Dichtlippe 18c bildet eine erste Dichtlippe 18c. Des Weiteren weist die Befestigungsvorrichtung 10c eine zweite den Unterdruckraum 16c teilweise begrenzende Dichtlippe 24c auf. Die Befestigungsvorrichtung 10c weist ferner zwei Rückstellelemente 20c, 20c' auf. Die Rückstellelemente 20c, 20c' sind mit jeweils einer der Dichtlippen 18c, 24c einstückig ausgebildet. Des Weiteren weist die Befestigungsvorrichtung 10c einen von dem Unterdruckraum 16c getrennten Funktionsraum 22c auf.

Die Befestigungsvorrichtung 10c weist ferner einen Grundkörper 26c auf. Der Grundkörper 26c der Befestigungsvorrichtung 10c weist ein Montageelement 28c auf. Das Montageelement 28c ist zu einer direkten Montage des Objekts 12c vorgesehen. Das Montageelement 28c des Grundkörpers 26c ist von einem Gewinde gebildet. Das Montageelement 28c ist von einem Innengewinde gebildet. Grundsätzlich wäre jedoch auch eine andere, einem Fachmann als sinnvoll erscheinende Ausbildung des Montageelements 28c denkbar. Es sind verschiedene, einem Fachmann als sinnvoll erscheinende Ausbildungen des Objekts 12c denkbar. Das Objekt 12c ist hier beispielhaft von einer Abdeckung mit einer Pumpe 40c gebildet. Das Objekt 12c grenzt direkt an den Funktionsraum 22c an. Das Objekt 12c begrenzt den Funktionsraum 22c in eine von der Oberfläche 14c angewandten Richtung. Ferner weist das Objekt 12c ein biegeschlaffes Abdeckelement 42c auf. Das Abdeckelement 42c ist von einer Membran gebildet. Ein Druck in dem Funktionsraum 22c kann eingestellt werden. Ein Druck in dem Funktionsraum 22c kann über die Pumpe 40c eingestellt werden. In dem Funktionsraum 22c kann sowohl ein Unter- als auch ein Überdruck eingestellt werden.

Eine weitere mögliche Anwendung der Befestigungsvorrichtung 10c liegt daher insbesondere in einer Version mit z.B. einem steifen Innenraum oder einer biegeschlaffen Abdeckung. In dem Funktionsraum 22c kann ein gewünschter Druck, z.B. Unterdruck, Überdruck oder Umgebungsdruck eingestellt werden.

Bei einem Verfahren zu einem Betrieb der Befestigungsvorrichtung 10c wird die Befestigungsvorrichtung 10c auf einer von einer Hautfläche gebildeten Oberfläche 14c befestigt.

Ferner kann mittels der Befestigungsvorrichtung 10c ein Muster auf der Oberfläche 14c erzeugt werden. Mittels der Befestigungsvorrichtung 10c kann ein Muster auf der Hautfläche erzeugt werden. Das Muster kann dabei sowohl von einem Sugillationsmuster gebildet sein, welches in dem Unterdruckraum 16c oder dem Funktionsraum 22c entsteht, als auch von einem Aufdruck mittels Farbe, welcher in dem Funktionsraum 22c aufgebracht wird. Dadurch kann insbesondere eine frei wählbare Lifestyle-Zeichnung auf der Haut und/oder einer anderen Oberfläche 14c erzeugt werden.

Grundsätzlich kann das Verfahren auch in alternativen Bereichen angewendet werden.

Figur 8 zeigt eine Befestigungsvorrichtung 10d. Die Befestigungsvorrichtung 10d ist zu einer Befestigung eines Objekts 12d an einer Oberfläche 14d vorgesehen. Die Befestigungsvorrichtung 10d ist zu einer kraftschlüssigen Befestigung eines Objekts 12d an einer Oberfläche 14d vorgesehen. Die Oberfläche 14d ist hier beispielhaft von einer Hautfläche gebildet.

Die Befestigungsvorrichtung 10d weist einen Unterdruckraum 16d auf. Der Unterdruckraum 16d grenzt in einem Befestigungszustand mit einer Seite an die Oberfläche 14d an. In einem nicht befestigten Zustand ist diese Seite des Unterdruckraums 16d geöffnet. Der Unterdruckraum 16d ist geometrisch unkonvex ausgebildet. Der Unterdruckraum 16d weist eine nichtkonvexe Menge auf. Der Unterdruckraum 16d weist eine C-förmige Grundform auf.

Ferner weist die Befestigungsvorrichtung 10d eine den Unterdruckraum 16d teilweise begrenzende Dichtlippe 18d auf. Die Dichtlippe 18d begrenzt den Unterdruckraum 16d zu einer Außenseiten hin. Die Dichtlippe 18d ist dazu vorgesehen, in dem Befestigungszustand an der Oberfläche 14d anzuliegen. Die Dichtlippe 18d ist umlaufend ausgebildet. Die Dichtlippe 18d ist dazu vorgesehen, den Unterdruckraum 16d an der Oberfläche 14d gegenüber einer Umgebung abzudichten. Die Dichtlippe 18d umschließt dafür den Unterdruckraum 16d in einer Ebene. Die Befestigungsvorrichtung 10d weist ferner einen Grundkörper 26d auf. Der Grundkörper 26d ist ebenfalls C-förmig ausgebildet. Die Dichtlippe 18d ist an dem Grundkörper 26d angeordnet. In einer Schnittebene senkrecht zu einer Haupterstreckungsebene 34d der Befestigungsvorrichtung 10d weist die Dichtlippe 18d eine konische, spitz zulaufende Form auf. Die Dichtlippe 18d bildet an einem freien, dem Grundkörper 26d abgewandten Ende eine Dichtkante. Die Dichtlippe 18d ist bei einer Montage dazu vorgesehen, durch Oberfläche 14d verspreizt zu werden. Die Dichtkante der Dichtlippe 18d liegt dabei ständig an der Oberfläche 14d an. Die Dichtlippe 18d ist in einem Befestigungszustand dazu vorgesehen, bündig an der Oberfläche 14d anzuliegen.

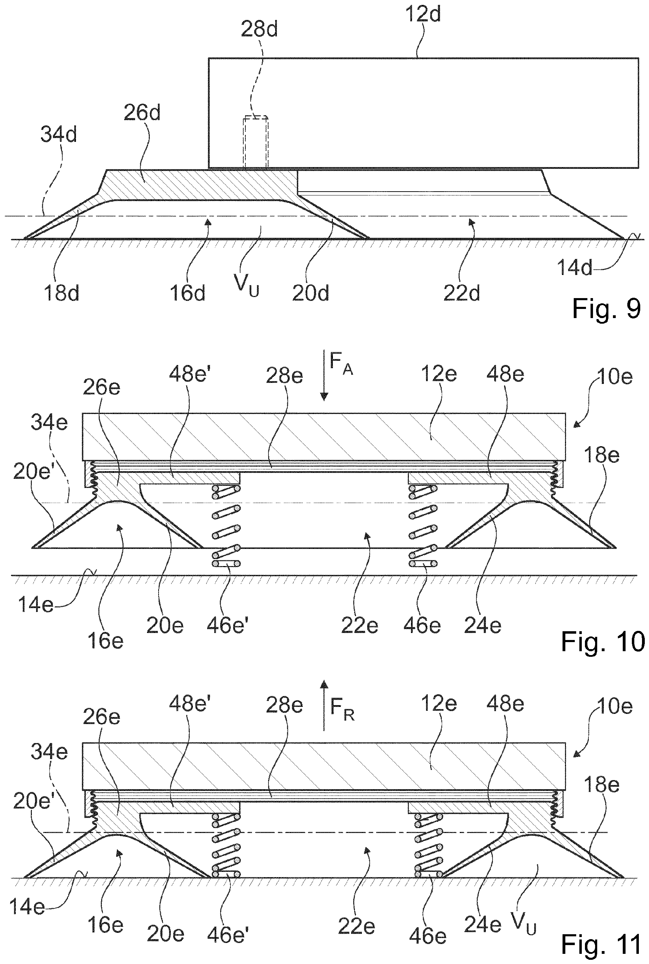

Ferner weist die Befestigungsvorrichtung 10d ein Rückstellelement 20d auf. Das Rückstellelement 20d ist dazu vorgesehen, in einem Befestigungszustand in dem Unterdruckraum 16d einen relativen Unterdruck zu erzeugen. Das Rückstellelement 20d ist dazu vorgesehen, in einem Befestigungszustand, zu einer Erzeugung des relativen Unterdrucks, ein Volumen V_{U} des abgeschlossenen Unterdruckraums 16d zu vergrößern. Das Rückstellelement 20d ist hierzu in einem Befestigungszustand dazu vorgesehen, eine von der Oberfläche 14d wegweisende Kraft auf die Befestigungsvorrichtung 10d zu wirken. Das Rückstellelement 20d ist einstückig mit der Dichtlippe 18d ausgebildet. Die Dichtlippe 18d ist derart elastisch ausgebildet, dass sie als Rückstellelement 20d fungiert. Die Dichtlippe 18d bildet das Rückstellelement 20d. Grundsätzlich wäre jedoch auch denkbar, dass das Rückstellelement 20d beispielsweise in die Dichtlippe 18d eingegossen ist, um eine vorteilhaft hohe Rückstellkraft F_{R} zu erreichen (Figur 9).

Des Weiteren weist die Befestigungsvorrichtung 10d einen von dem Unterdruckraum 16d getrennten Funktionsraum 22d auf. Der Funktionsraum 22d ist zumindest in einem Befestigungszustand von dem Unterdruckraum 16d getrennt. Der Funktionsraum 22d ist zu einer direkten Nutzung durch das zu befestigende Objekt 12d vorgesehen. Der Funktionsraum 22d ist zu einer direkten Bereitstellung eines Zugangs zu der Oberfläche 14d vorgesehen. Der Funktionsraum 22d ist teilweise von dem Unterdruckraum 16d umgeben. Der Funktionsraum 22d ist in einer Ebene parallel zu der Oberfläche 14d teilweise von dem Unterdruckraum 16d umgeben. Der Funktionsraum 22d wird in einer Ebene parallel zu einer Haupterstreckungsebene 34d der Befestigungsvorrichtung 10d, ausgehend von einem geometrischen Mittelpunkt des Funktionsraums 22d, in einem Winkelbereich von annähernd 180° von dem Unterdruckraum 16d umschlossen. Es existiert daher eine gedachte Gerade, welche entlang ihrer Erstreckung erst den Unterdruckraum 16d, anschließend den Funktionsraum 22d und zuletzt nochmals den Unterdruckraum 16d schneidet. Der Funktionsraum 22d ist in einem Befestigungszustand gegenüber dem Unterdruckraum 16d abgedichtet. Die Dichtlippe 18d ist in dem Befestigungszustand dazu vorgesehen, den Unterdruckraum 16d von dem Funktionsraum 22d zu trennen. Die Dichtlippe 18d ist zwischen dem Funktionsraum 22d und dem Unterdruckraum 16d angeordnet (Figur 8, 9).

Der Grundkörper 26d der Befestigungsvorrichtung 10d weist ein Montageelement 28d auf. Das Montageelement 28d ist zu einer direkten Montage des Objekts 12d vorgesehen. Das Montageelement 28d des Grundkörpers 26d ist von einer Gewindestange gebildet. Grundsätzlich wäre jedoch auch eine andere, einem Fachmann als sinnvoll erscheinende Ausbildung des Montageelements 28d denkbar.

Es sind verschiedene, einem Fachmann als sinnvoll erscheinende Ausbildungen des Objekts 12d denkbar.

Figur 10 zeigt eine Befestigungsvorrichtung 10e. Die Befestigungsvorrichtung 10e ist zu einer Befestigung eines Objekts 12e an einer Oberfläche 14e vorgesehen. Die Befestigungsvorrichtung 10e ist zu einer kraftschlüssigen Befestigung eines Objekts 12e an einer Oberfläche 14e vorgesehen.

Die Befestigungsvorrichtung 10e weist einen Unterdruckraum 16e auf. Der Unterdruckraum 16e ist geometrisch unkonvex ausgebildet. Der Unterdruckraum 16e weist eine nichtkonvexe Menge auf. Der Unterdruckraum 16e ist im Wesentlichen ringförmig ausgebildet. Ferner weist die Befestigungsvorrichtung 10e eine den Unterdruckraum 16e teilweise begrenzende Dichtlippe 18e auf. Die Dichtlippe 18e bildet eine erste Dichtlippe 18e. Des Weiteren weist die Befestigungsvorrichtung 10e eine zweite den Unterdruckraum 16e teilweise begrenzende Dichtlippe 24e auf. Die Dichtlippen 18e, 24e sind auf gegenüberliegenden Seiten des Unterdruckraums 16e angeordnet. Die Dichtlippen 18e, 24e sind dazu vorgesehen, durch die Oberfläche 14e in entgegengesetzte Richtungen elastisch verformt zu werden. Die Dichtlippen 18e, 24e sind jeweils bei einer Montage dazu vorgesehen, durch die Oberfläche 14e gegeneinander verspreizt zu werden.

Des Weiteren weist die Befestigungsvorrichtung 10e einen von dem Unterdruckraum 16e getrennten Funktionsraum 22e auf. Der Funktionsraum 22e ist in einer Ebene parallel zu der Oberfläche 14e vollständig von dem Unterdruckraum 16e umgeben.

Die Befestigungsvorrichtung 10e weist ferner vier Rückstellelemente 20e, 20e', 46e, 46e' auf. Die Rückstellelemente 20e, 20e', 46e, 46e' sind dazu vorgesehen, in einem Befestigungszustand in dem Unterdruckraum 16e einen relativen Unterdruck zu erzeugen. Zwei Rückstellelemente 20e, 20e' sind mit jeweils einer der Dichtlippen 18e, 24e einstückig ausgebildet. Die weiteren zwei Rückstellelemente 46e, 46e' sind jeweils von einem separaten Federelement gebildet. Die weiteren Rückstellelemente 46e, 46e' sind jeweils von einer Schraubenfeder gebildet. Die Ausbildung sowie die Dimensionierung der Federelemente in diesem Ausführungsbeispiel sind hier lediglich beispielhaft. Es sind grundsätzlich auch andere, einem Fachmann als sinnvoll erscheinende Ausbildungen und Dimensionierungen der Federelemente denkbar. Die weiteren Rückstellelemente 46e, 46e' erstrecken sich in einem Befestigungszustand im Wesentlichen senkrecht zu der Oberfläche 14e. Die weiteren Rückstellelemente 46e, 46e' ragen in einem entspannten Zustand über einen Rest der Befestigungsvorrichtung 10e heraus (Figur 10). Bei einer Montage werden die Rückstellelemente 46e, 46e' bei einem Aufbringen der Befestigungsvorrichtung 10e auf die Oberfläche 14e durch die Oberfläche 14e gestaucht und erzeugen jeweils eine Rückstellkraft F_{R} (Figur 11). Die weiteren zwei Rückstellelemente 46e, 46e' sind jeweils an einem Steg 48e, 48e' angeordnet. Die Stege 48e, 48e' sind an einem Grundkörper 26e der Befestigungsvorrichtung 10e angeordnet. Die Stege 48e, 48e' sind einstückig mit dem Grundkörper 26e ausgebildet. Die Stege 48e, 48e' ragen von gegenüberliegenden Seiten in den Funktionsraum 22e. Grundsätzlich wäre auch denkbar, dass die Befestigungsvorrichtung 10e lediglich die weiteren Rückstellelemente 46e, 46e' aufweist. Hierbei wäre beispielsweise denkbar, dass die Dichtlippen 18e, 24e bei einer Verformung keine Rückstellkraft erzeugen.

Der Grundkörper 26e der Befestigungsvorrichtung 10e weist ein Montageelement 28e auf. Das Montageelement 28e ist zu einer direkten Montage des Objekts 12e vorgesehen. Das Montageelement 28e des Grundkörpers 26e ist von einem Gewinde gebildet. Das Montageelement 28e ist von einem Außengewinde gebildet. Das Montageelement 28e bildet an einer Außenseite des Grundkörpers 26e das Gewinde aus. Das Objekt 12e kann mittels eines Innengewindes über das Montageelement 28e mit der Befestigungsvorrichtung 10e verbunden werden. Grundsätzlich wäre jedoch auch die Verwendung eines Außengewindes denkbar.

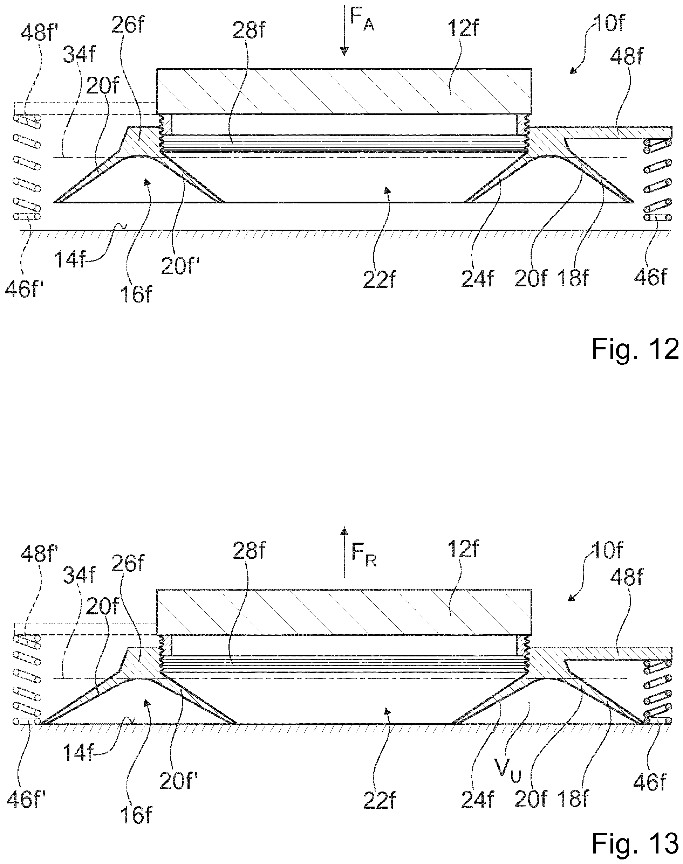

Es sind verschiedene, einem Fachmann als sinnvoll erscheinende Ausbildungen des Objekts 12e denkbar. Figur 12 zeigt eine Befestigungsvorrichtung 10f. Die Befestigungsvorrichtung 10f ist zu einer Befestigung eines Objekts 12f an einer Oberfläche 14f vorgesehen. Die Befestigungsvorrichtung 10f ist zu einer kraftschlüssigen Befestigung eines Objekts 12f an einer Oberfläche 14f vorgesehen.

Die Befestigungsvorrichtung 10f weist einen Unterdruckraum 16f auf. Der Unterdruckraum 16f ist geometrisch unkonvex ausgebildet. Der Unterdruckraum 16f weist eine nichtkonvexe Menge auf. Der Unterdruckraum 16f ist im Wesentlichen ringförmig ausgebildet. Ferner weist die Befestigungsvorrichtung 10f eine den Unterdruckraum 16f teilweise begrenzende Dichtlippe 18f auf. Die Dichtlippe 18f bildet eine erste Dichtlippe 18f. Des Weiteren weist die Befestigungsvorrichtung 10f eine zweite den Unterdruckraum 16f teilweise begrenzende Dichtlippe 24f auf. Die Dichtlippen 18f, 24f sind auf gegenüberliegenden Seiten des Unterdruckraums 16f angeordnet. Die Dichtlippen 18f, 24f sind dazu vorgesehen, durch die Oberfläche 14f in entgegengesetzte Richtungen elastisch verformt zu werden. Die Dichtlippen 18f, 24f sind jeweils bei einer Montage dazu vorgesehen, durch die Oberfläche 14f gegeneinander verspreizt zu werden.

Des Weiteren weist die Befestigungsvorrichtung 10f einen von dem Unterdruckraum 16f getrennten Funktionsraum 22f auf. Der Funktionsraum 22f ist in einer Ebene parallel zu der Oberfläche 14f vollständig von dem Unterdruckraum 16f umgeben.

Die Befestigungsvorrichtung 10f weist ferner vier Rückstellelemente 20f, 20f', 46f, 46f' auf. Die Rückstellelemente 20f, 20f', 46f, 46f' sind dazu vorgesehen, in einem Befestigungszustand in dem Unterdruckraum 16f einen relativen Unterdruck zu erzeugen. Zwei Rückstellelemente 20f, 20f' sind mit jeweils einer der Dichtlippen 18f, 24f einstückig ausgebildet. Die weiteren zwei Rückstellelemente 46f, 46f' sind jeweils von einem separaten Federelement gebildet. Die weiteren Rückstellelemente 46f, 46f' sind jeweils von einer Schraubenfeder gebildet. Die weiteren Rückstellelemente 46f, 46f' erstrecken sich in einem Befestigungszustand im Wesentlichen senkrecht zu der Oberfläche 14f. Die weiteren Rückstellelemente 46f, 46f' ragen in einem entspannten Zustand über einen Rest der Befestigungsvorrichtung 10f heraus (Figur 12). Bei einer Montage werden die Rückstellelemente 46f, 46f' bei einem Aufbringen der Befestigungsvorrichtung 10f auf die Oberfläche 14f durch die Oberfläche 14f gestaucht und erzeugen jeweils eine Rückstellkraft F_{R} (Figur 13). Eines der weiteren Rückstellelemente 46f ist an einem Steg 48f angeordnet. Der Steg 48f ist an dem Grundkörper 26f der Befestigungsvorrichtung 10f angeordnet. Der Steg 48f ist einstückig mit dem Grundkörper 26f ausgebildet. Der Steg 48f ragt in eine Umgebung der Befestigungsvorrichtung 10f. Der Steg 48f ragt in eine von dem Funktionsraum 22f wegweisende Richtung aus dem Grundkörper 26f der Befestigungsvorrichtung 10f. Grundsätzlich wäre jedoch auch denkbar, dass zumindest eines der Rückstellelemente 46f' an dem Objekt 12f befestigt ist, wie dies gestrichelt angedeutet ist. Hierzu ist das Rückstellelement 46f' an einem Steg 48f' angeordnet. Der Steg 48f' ist an dem Objekt 12f angeordnet. Der Steg 48f' ragt in eine Umgebung des Objekts 12f. Der Steg 48f' ragt in eine von dem Funktionsraum 22f wegweisende Richtung aus dem Objekt 12f. Die beiden Rückstellelemente 46f, 46f' sind hier beispielhaft in Kombination gezeigt. Grundsätzlich wäre jedoch auch eine getrennte Anwendung der Rückstellelemente 46f, 46f' denkbar.

Es sind verschiedene, einem Fachmann als sinnvoll erscheinende Ausbildungen des Objekts 12f denkbar.

## Patentansprüche

1. Befestigungsvorrichtung zu einer Befestigung eines Objekts (12a; 12b; 12c; 12d; 12e; 12f) an einer Oberfläche (14a; 14b; 14c; 14d; 14e; 14f), mit zumindest einem Unterdruckraum (16a; 16b; 16c; 16d; 16e; 16f), welcher dazu vorgesehen ist, in einem Befestigungszustand an die Oberfläche (14a; 14b; 14c; 14d; 14e; 14f) anzugrenzen, mit zumindest einer den Unterdruckraum (16a; 16b; 16c; 16d; 16e; 16f) zumindest teilweise begrenzenden Dichtlippe (18a; 18b; 18c; 18d; 18e; 18f), welche dazu vorgesehen ist, in dem Befestigungszustand an der Oberfläche (14a; 14b; 14c; 14d; 14e; 14f) anzuliegen, und mit zumindest einem Rückstellelement (20a, 20a'; 20b, 20b'; 20c, 20c'; 20d; 20e, 20e', 46e, 46e'; 20f, 20f', 46f, 46f'), welches dazu vorgesehen ist, in einem Befestigungszustand in dem Unterdruckraum (16a; 16b; 16c; 16d; 16e; 16f) einen relativen Unterdruck zu erzeugen, wobei die Befestigungsvorrichtung zumindest einen von dem Unterdruckraum (16a; 16b; 16c; 16d; 16e; 16f) getrennten Funktionsraum (22a; 22b; 22c; 22d; 22e; 22f) aufweist, welcher zumindest teilweise von dem Unterdruckraum (16a; 16b; 16c; 16d; 16e; 16f) umgeben ist und dazu vorgesehen ist, in einem Befestigungszustand an die Oberfläche (14a; 14b; 14c; 14d; 14e; 14f) anzugrenzen,
**dadurch gekennzeichnet, dass**
das zumindest eine Rückstellelement (20a, 20a'; 20b, 20b'; 20c, 20c'; 20d; 20e, 20e'; 20f, 20f') einstückig mit der zumindest einen Dichtlippe (18a, 24a; 18b, 24b; 18c, 24c; 18d; 18e; 18f) ausgebildet ist, wobei die zumindest eine Dichtlippe (18a, 24a; 18b, 24b; 18c, 24c; 18d; 18e; 18f) das zumindest eine Rückstellelement (20a, 20a'; 20b, 20b'; 20c, 20c'; 20d; 20e, 20e'; 20f, 20f') ausbildet.

2. Befestigungsvorrichtung nach Anspruch 1,
**dadurch gekennzeichnet, dass**
der zumindest eine Unterdruckraum (16a; 16b; 16c; 16d; 16e; 16f) geometrisch unkonvex ausgebildet ist.

3. Befestigungsvorrichtung nach Anspruch 1 oder 2,
**gekennzeichnet durch**
zumindest eine zweite den Unterdruckraum (16a; 16b; 16c; 16e; 16f) zumindest teilweise begrenzende Dichtlippe (24a; 24b; 24c; 24e; 24f), welche von der zumindest einen Dichtlippe (18a; 18b; 18c; 18e; 18f) differierend ausgebildet ist und dazu vorgesehen ist, in dem Befestigungszustand an der Oberfläche (14a; 14b; 14c; 14e; 14f) anzuliegen.

4. Befestigungsvorrichtung nach Anspruch 3,
**dadurch gekennzeichnet, dass**
die zumindest eine zweite Dichtlippe (24a; 24b; 24c; 24e; 24f) in dem Befestigungszustand dazu vorgesehen ist, den zumindest einen Unterdruckraum (16a; 16b; 16c; 16e; 16f) von dem zumindest einen Funktionsraum (22a; 22b; 22c; 22e; 22f) zu trennen.

5. Befestigungsvorrichtung nach einem der vorhergehenden Ansprüche,
**dadurch gekennzeichnet, dass**
der zumindest eine Funktionsraum (22a; 22b; 22c; 22e; 22f) in einem Befestigungszustand gegenüber einer Umgebung abgedichtet ist.

6. Befestigungsvorrichtung nach Anspruch 5,
**dadurch gekennzeichnet, dass**
ein Druck in dem zumindest einen Funktionsraum (22c) eingestellt werden kann.

7. Befestigungsvorrichtung nach einem der vorhergehenden Ansprüche,
**gekennzeichnet durch**
einen Grundkörper (26a; 26b; 26c; 26d; 26e; 26f), an welchem die zumindest eine Dichtlippe (18a, 24a; 18b, 24b; 18c, 24c; 18d; 18e, 24e; 18f, 24f) angeordnet ist und welche zumindest ein Montageelement (28a; 28b; 28c; 28d; 28e; 28f) zu einer direkten Montage des Objekts (12a; 12b; 12c; 12d; 12e; 12f) aufweist.

8. Befestigungsvorrichtung nach Anspruch 7,
**dadurch gekennzeichnet, dass**
das zumindest eine Montageelement (28a; 28b; 28c; 28d; 28e; 28f) des Grundkörpers (26a; 26b; 26c; 26d; 26e; 26f) zumindest teilweise von einem Gewinde gebildet ist.

9. Verfahren zu einer Montage und/oder einem Betrieb der Befestigungsvorrichtung (10a; 10b; 10c; 10d; 10e; 10f) nach einem der vorhergehenden Ansprüche.

10. Verfahren nach Anspruch 9,
**dadurch gekennzeichnet, dass**
die Befestigungsvorrichtung (10a; 10b; 10c; 10d; 10e; 10f) in einem ersten Schritt (30a) mittels einer Kraft (F_{A}) gegen die Oberfläche (14a; 14b; 14c; 14d; 14; 14f) gedrückt wird, sodass sich ein Volumen (Vu) des zumindest einen Unterdruckraums (16a; 16b; 16c; 16d; 16e; 16f) verringert und das zumindest eine Rückstellelement (20a, 20a'; 20b, 20b'; 20c, 20c'; 20d; 20e, 20e', 46e, 46e'; 20f, 20f', 46f, 46f') ausgelenkt wird.

11. Verfahren nach Anspruch 10,
**dadurch gekennzeichnet, dass**
das zumindest eine Rückstellelement (20a, 20a'; 20b, 20b'; 20c, 20c'; 20d; 20e, 20e', 46e, 46e'; 20f, 20f', 46f, 46f') in einem weiteren Schritt (32a) eine entgegen der Kraft (F_{A}) gerichtete Rückstellkraft (F_{R}) erzeugt, welche einen relativen Unterdruck in dem zumindest einen Unterdruckraum (16a; 16b; 16c; 16d; 16e; 16f) erzeugt.

12. Verfahren nach einem der Ansprüche 9 bis 11,
**dadurch gekennzeichnet, dass**
die Durchführung unter Bedingungen reduzierter Schwerkraft erfolgt.

13. Verfahren nach einem der Ansprüche 9 bis 12,
**dadurch gekennzeichnet, dass**
die Oberfläche (14b; 14c; 14d) von einer Hautfläche gebildet ist.

14. Verfahren nach Anspruch 13,
**dadurch gekennzeichnet, dass**
mittels der Befestigungsvorrichtung (10b; 10c; 10d) ein Muster auf der Oberfläche (14b; 14c; 14d) erzeugt wird.

15. Verfahren nach einem der Ansprüche 9 bis 14,
**dadurch gekennzeichnet, dass**
in einem Verfahrensschritt auf einen einen Funktionsraum (22b) begrenzenden Bereich (44b) der Oberfläche (14b) eine Substanz (38b) auf die Oberfläche (14b) aufgebracht wird.

16. Verfahren nach Anspruch 15,
**dadurch gekennzeichnet, dass**
in einem Verfahrensschritt die auf den, den Funktionsraum (22b) begrenzenden Bereich (44b) der Oberfläche (14b) aufgebrachte Substanz (38b) mittels eines Objekts (12b) gemessen wird.

## Claims

1. Fixation device for a fixation of an object (12a; 12b; 12c; 12d; 12e; 12f) on a surface (14a; 14b; 14c; 14d; 14e; 14f),
with at least one underpressure space (16a; 16b; 16c; 16d; 16e; 16f) which is configured to adjoin the surface (14a; 14b; 14c; 14d; 14e; 14f) in a fixation state,
with at least one sealing lip (18a; 18b; 18c; 18d; 18e; 18f) which at least partially delimits the underpressure space (16a; 16b; 16c; 16d; 16e; 16f) and which is configured to adjoin the surface (14a; 14b; 14c; 14d; 14e; 14f) in the fixation state,
and with at least one reset element (20a, 20a'; 20b, 20b'; 20c; 20c'; 20d; 20e, 20e', 46e, 46e'; 20f, 20f', 46f, 46f') which is configured to generate in a fixation state a relative underpressure in the underpressure space (16a; 16b; 16c; 16d; 16e; 16f),
wherein the fixation device comprises at least one functional space (22a; 22b; 22c; 22d; 22e; 22f) which is separate from the underpressure space (16a; 16b; 16c; 16d; 16e; 16f), which is encompassed at least partially by the underpressure space (16a; 16b; 16c; 16d; 16e; 16f) and which is configured to adjoin the surface (14a; 14b; 14c; 14d; 14e; 14f) in a fixation state,
**characterised in that** the at least one reset element (20a, 20a'; 20b, 20b'; 20c; 20c'; 20d; 20e, 20e'; 20f, 20f') is embodied integrally with the at least one sealing lip (18a, 24a; 18b, 24b; 18c, 24c; 18d; 18e; 18f),
wherein the at least one sealing lip (18a, 24a; 18b, 24b; 18c, 24c; 18d; 18e; 18f) forms the at least one reset element (20a, 20a'; 20b, 20b'; 20c; 20c'; 20d; 20e, 20e'; 20f, 20f').

2. Fixation device according to claim 1,
**characterised in that** the at least one underpressure space (16a; 16b; 16c; 16d; 16e; 16f) is embodied to be geometrically non-convex.

3. Fixation device according to claim 1 or 2,
**characterised by** at least one second sealing lip (24a; 24b; 24c; 24e; 24f) which at least partially delimits the underpressure space (16a; 16b; 16c; 16e; 16f) and which is embodied differently than the at least one sealing lip (18a; 18b; 18c; 18e; 18f) and which is configured to adjoin the surface (14a; 14b; 14c; 14e; 14f) in the fixation state.

4. Fixation device according to claim 3,
**characterised in that** in the fixation state the at least one second sealing lip (24a; 24b; 24c; 24e; 24f) is configured to separate the at least one underpressure space (16a; 16b; 16c; 16e; 16f) from the at least one functional space (22a; 22b; 22c; 22e; 22f).

5. Fixation device according to one of the preceding claims,
**characterised in that** in a fixation state the at least one functional space (22a; 22b; 22c; 22e; 22f) is sealed against an environment.

6. Fixation device according to claim 5,
**characterised in that** a pressure is adjustable in the at least one functional space (22c).

7. Fixation device according to one of the preceding claims,
**characterised by** a base body (26a; 26b; 26c; 26d; 26e; 26f) which the at least one sealing lip (18a, 24a; 18b, 24b; 18c, 24c; 18d; 18e, 24e; 18f, 24f) is arranged on and which comprises at least one mounting element (28a; 28b; 28c; 28d; 28e; 28f) for a direct mounting of the object (12a; 12b; 12c; 12d; 12e; 12f).

8. Fixation device according to claim 7,
**characterised in that** the at least one mounting element (28a; 28b; 28c; 28d; 28e; 28f) of the base body (26a; 26b; 26c; 26d; 26e; 26f) is implemented at least partly of a thread.

9. Method for a mounting and/or an operation of the fixation device (10a; 10b; 10c; 10d; 10e; 10f) according to one of the preceding claims.

10. Method according to claim 9,
**characterised in that** in a first step (30a) the fixation device (10a; 10b; 10c; 10d; 10e; 10f) is pressed against the surface (14a; 14b; 14c; 14d; 14e; 14f) by a force (F_{A}) such that a Volume (V_{U}) of the at least one underpressure space (16a; 16b; 16c; 16d; 16e; 16f) decreases and the at least one reset element (20a, 20a'; 20b, 20b'; 20c; 20c'; 20d; 20e, 20e', 46e, 46e'; 20f, 20f', 46f, 46f') is deflected.

11. Method according to claim 10,
**characterised in that** in a further step (32a) the at least one reset element (20a, 20a'; 20b, 20b'; 20c; 20c'; 20d; 20e, 20e', 46e, 46e'; 20f, 20f', 46f, 46f') generates a reset force (F_{R}) that is directed against the force (F_{A}) and induces a relative underpressure in the at least one underpressure space (16a; 16b; 16c; 16d; 16e; 16f).

12. Method according to one of claims 9 to 11,
**characterised in that** the implementation is effected under reduced-gravity conditions.

13. Method according to one of claims 9 to 12,
**characterised in that** the surface (14b; 14c; 14d) is embodied by a skin surface.

14. Method according to claim 13,
**characterised in that** a pattern is created on the surface (14b; 14c; 14d) by means of the fixation device (10b; 10c; 10d).

15. Method according to one of claims 9 to 14,
**characterised in that** in one method step a substance (38b) is applied to the surface (14b), onto a region (44b) of the surface (14b) that delimits a functional space (22b).

16. Method according to claim 15,
**characterised in that** in one method step the substance (38b) applied onto the region (44b) of the surface (14b) that delimits the functional space (22b) is measured by means of an object (12b).

## Revendications

1. Dispositif de fixation pour une fixation d'un objet (12a ; 12b ; 12c ; 12d ; 12e ; 12f) sur une surface (14a ; 14b ; 14c ; 14d ; 14e ; 14f),
avec au moins un espace de dépressurisation (16a ; 16b ; 16c ; 16d ; 16e ; 16f) qui est prévu pour jouxter la surface (14a ; 14b ; 14c ; 14d ; 14e ; 14f) dans un état de fixation,
avec au moins une lèvre d'étanchéité ((18a ; 18b ; 18c ; 18d ; 18e ; 18f) qui délimite au moins partiellement l'espace de dépressurisation (16a ; 16b ; 16c ; 16d ; 16e ; 16f) et est prévue pour être adjacente à la surface (14a ; 14b ; 14c ; 14d ; 14e ; 14f) dans l'état de fixation,
et avec au moins un élément de rappel (20a, 20a' ; 20b, 20b' ; 20c, 20c' ; 20d ; 20e, 20e', 46e, 46e' ; 20f, 20f', 46f, 46f') prévu pour générer, dans un état de fixation, une dépressurisation relative dans l'espace de dépressurisation (16a ; 16b ; 16c ; 16d ; 16e ; 16f),
le dispositif de fixation comprenant au moins un espace fonctionnel (22a ; 22b ; 22c ; 22d ; 22e ; 22f) qui est séparé de l'espace de dépressurisation (16a ; 16b ; 16c ; 16d ; 16e ; 16f), qui est entouré au moins partiellement de l'espace de dépressurisation (16a ; 16b ; 16c ; 16d ; 16e ; 16f) et qui est prévu pour jouxter la surface (14a ; 14b ; 14c ; 14d ; 14e ; 14f) dans un état de fixation,
**caractérisé en ce que** l'au moins un élément de rappel (20a, 20a' ; 20b, 20b' ; 20c, 20c' ; 20d ; 20e, 20e' ; 20f, 20f') est réalisé intégralement avec l'au moins une lèvre d'étanchéité (18a, 24a ; 18b, 24b ; 18c, 24c ; 18d ; 18e ; 18f),
l'au moins une lèvre d'étanchéité (18a, 24a ; 18b, 24b ; 18c, 24c ; 18d ; 18e ; 18f) formant l'au moins un élément de rappel (20a, 20a' ; 20b, 20b' ; 20c, 20c' ; 20d ; 20e, 20e' ; 20f, 20f').

2. Dispositif de fixation selon la revendication 1,
**caractérisé en ce que** l'au moins un espace de dépressurisation (16a ; 16b ; 16c ; 16d ; 16e ; 16f) est implémenté d'une manière géométriquement non-convexe.

3. Dispositif de fixation selon la revendication 1 ou 2,
**caractérisé par** au moins une deuxième lèvre d'étanchéité (24a ; 24b ; 24c ; 24e ; 24f) qui délimite au moins partiellement l'espace de dépressurisation (16a ; 16b ; 16c ; 16e ; 16f), qui est implémentée différente de l'au moins une lèvre d'étanchéité (18a ; 18b ; 18c ; 18e ; 18f) et qui est prévue pour jouxter la surface (14a ; 14b ; 14c ; 14e ; 14f) dans l'état de fixation.

4. Dispositif de fixation selon la revendication 3,
**caractérisé en ce que** l'au moins une deuxième lèvre d'étanchéité (24a ; 24b ; 24c ; 24e ; 24f) est prévue, dans l'état de fixation, pour séparer l'au moins un espace de dépressurisation (16a ; 16b ; 16c ; 16e ; 16f) de l'au moins un espace fonctionnel (22a ; 22b ; 22c ; 22e ; 22f)..

5. Dispositif de fixation selon l'une quelconque des revendications précédentes,
**caractérisé en ce que** l'au moins un espace fonctionnel (22a ; 22b ; 22c ; 22e ; 22f) est scellé contre un environnement dans un état de fixation.

6. Dispositif de fixation selon la revendication 5,
**caractérisé en ce qu'**une pression peut être ajustée dans l'au moins un espace fonctionnel (22c).

7. Dispositif de fixation selon l'une quelconque des revendications précédentes,
**caractérisé par** un corps de base (26a ; 26b ; 26c ; 26d ; 26e ; 26f), sur lequel l'au moins une lèvre d'étanchéité (18a, 24a ; 18b, 24b ; 18c, 24c ; 18d ; 18e, 24e ; 18f, 24f) est disposée et qui comporte au moins un élément de montage (28a ; 28b ; 28c ; 28d ; 28e ; 28f) pour un montage direct de l'objet (12a ; 12b ; 12c;12d; 12e; 12f)..

8. Dispositif de fixation selon la revendication 7,
**caractérisé en ce que** l'au moins un élément de montage (28a ; 28b ; 28c ; 28d ; 28e ; 28f) du corps de base (26a ; 26b ; 26c ; 26d ; 26e ; 26f) est implémenté au moins partiellement par un filetage.

9. Procédé pour montage et/ou opération du dispositif de fixation (10a ; 10b ; 10c ; 10d ; 10e ; 10f) selon l'une quelconque des revendications précédentes.

10. Procédé selon la revendication 9,
**caractérisé en ce que** dans une première étape (30a) le dispositif de fixation (10a ; 10b ; 10c ; 10d ; 10e ; 10f) est poussé contre la surface (14a ; 14b ; 14c ; 14d ; 14e ; 14f) par le biais d'une force (F_{A}) de sorte qu'un volume (V_{U}) de l'au moins un espace de dépressurisation (16a ; 16b ; 16c ; 16d ; 16e ; 16f) se réduise et l'au moins un élément de rappel (20a, 20a' ; 20b, 20b' ; 20c, 20c' ; 20d ; 20e, 20e', 46e, 46e' ; 20f, 20f', 46f, 46f') soit défléchi.

11. Procédé selon la revendication 10,
**caractérisé en ce que** dans une étape de plus (32a) l'au moins un élément de rappel (20a, 20a' ; 20b, 20b' ; 20c, 20c' ; 20d ; 20e, 20e', 46e, 46e' ; 20f, 20f', 46f, 46f') génère une force de rappel (F_{R}) portant contre la force (F_{A}), qui produit une dépressurisation relative dans l'au moins un espace de dépressurisation (16a ; 16b ; 16c ; 16d ; 16e ; 16f).

12. Procédé selon l'une des revendications 9 à 11,
**caractérisé en ce que** l'implémentation s'effectue sous les conditions de gravitation réduite.

13. Procédé selon l'une des revendications 9 à 12,
**caractérisé en ce que** la surface (14b ; 14c ; 14d) est réalisée comme une surface de peau.

14. Procédé selon la revendication 13,
**caractérisé en ce qu'**un motif est créé sur la surface (14b ; 14c ; 14d) par le dispositif de fixation (10b ; 10c ; 10d).

15. Procédé selon l'une des revendications 9 à 14,
**caractérisé en ce que** dans une étape du procédé une substance (38b) est appliquée sur la surface (14b), sur une zone (44b) de la surface (14b) qui délimite un espace fonctionnel (22b).

16. Procédé selon la revendication 15,
**caractérisé en ce que** dans une étape du procédé la substance (38b) appliquée sur la zone (44b) de la surface (14b) qui délimite l'espace fonctionnel (22b) est mesurée par le biais d'un objet (12b).
